# EUROPEAN PATENT APPLICATION

(11) **EP 2 924 037 A1**
(43) Date of publication of application: **30.09.2015**
(21) Application number: 12871851.7
(22) Date of filing: 23.03.2012
(51) Int. Cl.: C07D 413/14, A61K 31/517, A61P 1/00, A61P 1/16, A61P 3/10, A61P 7/02, A61P 9/00, A61P 9/10, A61P 11/00, A61P 13/12, A61P 31/00, A61P 31/04

(54) **QUINAZOLINE DERIVATE AND USE THEREOF AS APOPTOSIS INHIBITOR**

(71) Applicant: Linx Pharmaceutical Co., Ltd., Wuxi, Jiangsu 214092 (CN)
(72) Inventor: LIU, Lei, Wuxi, Jiangsu 214092 (CN); XU, Xiaochun, Wuxi, Jiangsu 214092 (CN); LI, Xiaoyu, Wuxi, Jiangsu 214092 (CN)
(74) Representative: Gulde & Partner
(86) International application number: PCT/CN2012/000363
(87) International publication number: WO 2013/138951

(57) **Abstract**

The present invention relates to the new use and target of a compound of formula I such as terazosin. In particular, the present invention relates to the use of the compound of formula I in the preparation of drugs used as an apoptosis inhibitor or drugs for treating and/or preventing sepsis and the complications thereof. The present invention provides a new method and direction for inhibiting apoptosis and treating and/or preventing sepsis and the complications thereof.

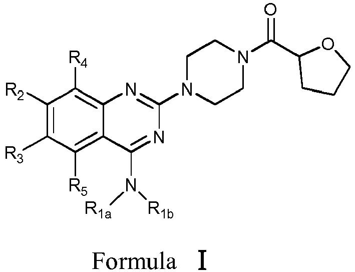

## Description

### TECHNICAL FIELD

The present invention relates to quinazoline derivatives and novel uses of quinazoline derivatives such as terazosin. In particular, the present invention relates to uses of quinazoline derivatives such as terazosin as an apoptosis inhibitor, for treating and/or preventing sepsis and its complications, and for activating new targets.

### BACKGROUND

Sepsis is the first cause of death in intensive care units all over the world. Sepsis is believed to be a complex modulating disorder of inflammation (Bone et al., 1996). However, many clinical trials of cytokines or specific anti-inflammatory drugs have not significantly improved the survival of sepsis patients, therefore, there remains a need to reconsider the strategy of treating sepsis. Though the effect is limited, treating sepsis with antibiotics is an important method. Basic researches discovered that experimental sepsis could be effectively blocked by inhibiting apoptosis. In sepsis patients, apoptosis of immune cells may further weaken immune responses. In addition, inhibiting cell apoptosis was proved to be an important strategy for treating sepsis (Braun et al., 1999; Hotchkiss et al., 2000; Chung et al., 2001; Weaver et al., 2004; Wesche-Solsato et al., 2005).

Apoptosis is a basic biological phenomenon of cells, which plays an essential role in multicellular organisms to remove unneeded or abnormal cells, and is important in evolution, homeostasis, and the development of multiple systems. Apoptosis is not only a special type of cell death, but also has biological significance and a complex molecular mechanism. Apoptosis is a strictly controlled process of multiple genes. These genes are conserved among different species, such as the Bcl-2 family, caspase family, oncogenes such as C-myc, tumor suppressor genes P53, etc. The development of molecular biology techniques has helped to gain considerable knowledge of the process of apoptosis, but so far the exact mechanism of apoptosis is not fully understood. Disorders in the process of apoptosis may have a direct or indirect relationship with many diseases. It is known that brain death caused by cerebral thrombosis is associated with apoptosis. Additionally, it is believed that activation of phosphoglycerate kinase 1 (pgk1) is helpful for treatment or prevention of some diseases related to apoptosis.

Terazosin is commonly used clinically in the hydrochloride form. The unit dosage forms of launched tablets and capsules include 1mg, 2mg, and 5mg. Hydrochloride terazosin can be used for treatment of benign prostatic hyperplasia, and can also be used alone or in combination with another blood pressure medication, such as diuretics or alpha-adrenergic blockers, to treat high blood pressure. In current clinical indications, usual adult daily dose of terazosin is 1-10mg. Terazosin can be used for treatment of benign prostatic hyperplasia (BPH). The reduction in symptoms of BPH and improvement in urine flow rates following administration of terazosin is related to relaxation of smooth muscles produced by alpha-adrenergic blockers in the bladder neck and prostate. Because there are relatively few alpha-adrenoceptors in the bladder body, terazosin is able to reduce the bladder outlet obstruction without affecting bladder contractility. Additionally, terasozin can reduce the peripheral vascular resistance to lower the blood pressure. Terazosin works by blocking alpha-adrenoceptors to relax blood vessels and lower blood pressure.

So far, there is still lack of effective apoptosis inhibitors. It is a remarkable research target to develop new anti-apoptotic compounds of anti-apoptosis for use in treatment and/or prevention of sepsis and its complications, such as sepsis of cerebral apoplexy and its complications.

### SUMMARY OF THE INVENTION

The present invention relates to new methods and targets in the treatment and/or prevention of sepsis and its complications. The present inventors unexpectedly discovered that a class of quinazoline derivatives, such as terazosin for treating BPH and high blood pressure clinically, can be used alone or in combination with antibiotics to effectively treat and/or prevent sepsis and its complications.

In a first aspect, the present invention relates to the compound of Formula I, or a pharmaceutical acceptable salt, solvate, ester, or prodrug thereof: 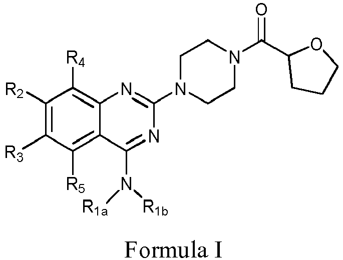 wherein,
R₁ₐ and R_{1b} are independently selected from H, NH₂, OH, C₁₋₆ alkyl, C₁₋₆ alkoxy -C₁₋₆ alkyl-, C₂₋₆ alkenyl, C₂₋₆ alkynyl, C₁₋₆ alkoxy, C₁₋₆a lkanoyl, aroyl, C₆₋₁₀ aryl, and C₅₋₆ cycloalkyl, or R₁ₐ and R_{1b} together with the nitrogen atom to which they are attached form a 5- or 6-membered ring, wherein said alkyl is optionally substituted with 1 to 3 substituents each independently selected from hydroxyl and a halogen;
R₂ and R₃ are independently selected from H, a halogen, C₁₋₆ alkyl, C₁₋₆ haloalkyl, C₂₋₆ alkenyl, C₂₋₆ alkynyl, CN, NO₂, NH₂, OH, C₁₋₆ alkoxy, C₁₋₆ alkoxy-C₁₋₆ alkoxy, C₁₋₆ alkanoyloxy, C₁₋₆ alkanoylamino, aroylamino, and saturated or unsaturated 5- or 6- membered carbocyclyl or heterocyclyl, C₁₋₆ alkanoyl, or R₂ and R₃ together with the ring atoms to which they are attached may form a 5- or 6- membered carbocyclic or heterocyclic ring; and
R₄ and R₅ are independently selected from H, a halogen, CN, NO₂, NH₂, OH, C₁₋₆ alkyl, C₁₋₆ alkoxy-C₁₋₆ alkoxy, C₁₋₆ alkanoyloxy, C₁₋₆ haloalkyl, C₂₋₆ alkenyl, C₂₋₆ alkynyl, C₁₋₆ alkoxy, C₁₋₆ alkanoylamino, aroylamino, saturated or unsaturated 5- or 6- membered carbocyclyl or heterocyclyl, saturated or unsaturated 5- or 6- membered carbocyclyloxy or heterocyclyloxy, or C₁₋₆ alkanoyl.

In a second aspect, the present invention relates to uses of the compound of Formula I, e.g., terazosin, in preparation of medicament as apoptosis inhibitors and/or pgk1 activators, or in preparation of medicament for the treatment and/or prevention of sepsis and the complications thereof.

In a third aspect, the present invention provides a pharmaceutical composition, which comprises a therapeutically and/or prophylactically effective amount of the compound of formula I, e.g. terazosin, or a pharmaceutically acceptable salt or solvate thereof, and optionally a pharmaceutically acceptable carrier.

In a fourth aspect, the present invention provides a kit, comprising a therapeutically and/or prophylactically effective amount of the compound of formula I, e.g. terazosin, or a pharmaceutically acceptable salt or solvate thereof, and at least one anti-microbial agent.

In a fifth aspect, the present invention relates to a method of inhibition of apoptosis in a subject in need thereof or in a biological sample, a method of activating pgk1 in a subject in need thereof or in a biological sample, or a method for the treatment and/or prevention of sepsis and its complications in a subject in need thereof.

In a sixth aspect, the present invention relates to the compound of formula I, e.g. terazosin, for use as an apoptosis inhibitor, a pgk1 activator, or for the treatment and/or prevention of sepsis and its complications.

### BRIEF DESCRIPTION OF THE DRAWINGS

Figure 1: shows the survival rate of apoptotic *HS>rpr* Drosophila after the induced expression of *HS-Gal4* for 1-3 hours. Each point represents the mean +SE (standard error). Test n = 6.
Figure 2: shows the list of compounds for drug screening. These compounds are selected based on Cmap and grouped according to the positive or negative correlation between their apoptosis microarrays.
Figure 3: shows drug screening using the apoptotic Drosophila model. The survival rate of apoptotic Drosophila administered with each drug is expressed as mean +SE. Control (no drug administered) is shown with a white bar. Different drugs are shown with white or black bars. Test n = 5.
Figure 4: shows that terazosin (4µg/ml) significantly blocked apoptosis caused by LPS- and IFNγ-induced cytotoxicity. Apoptotic cells were stained with Annexin V. Statistical test results show that terazosin can inhibit apoptosis. The number of cells counted in each group was 200-250.
Figure 5: Figure 5a shows the survival curve of the effect of terazosin (0.4mg/kg) on an LPS-induced sepsis model. The survival curve was analyzed by the Kaplan-Merier test using the Log Rank algorithm. P value and the number of tested mice are shown in the figure. Figure 5b shows DNA fragmentation on an agarose gel after LPS treatment. Each band represents a genomic DNA derived from mouse thymus. Treatment of each mouse is marked on top of each band.
Figure 6: shows the survival rate of mice administered with terazosin (0.04 mg / kg) after 12 hours after LPS injection (13.5mg/kg). It was analyzed by the Kaplan-Merier test with the Log Rank algorithm. P value and the number of tested mice are shown in the figure, and T in the figure represents terazosin.
Figure 7: shows the survival curve of a murine model ofE. coli sepsis. After 1.5 hours following injection of E. coli, terazosin (0.4 mg / kg) was administered. It was analyzed by the Kaplan-Merier test with the Log Rank algorithm. P value and the number of tested mice are shown in the figure.
Figure 8: illustrates the effects of terazosin on growth of E. coli. After 24 hours following terazosin application, the effects of terazosin and ampicillin on the growth of the bacteria were compared by examining the inhibition zones. The results show that the control (no drug added) and different doses of terazosin (0.2µg T, 2µg T, 200µgT, 2mg T) cannot inhibit the growth of E. coli, but ampicillin (2mg) is shown to inhibit E. coli growth.

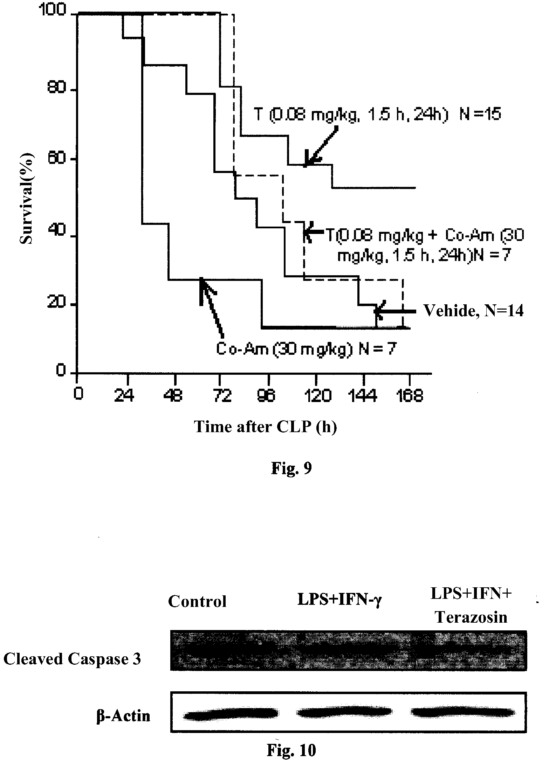

Figure 9: shows the survival curves of the role of terazosin on a murine model of CLP-induced sepsis. Terazosin was injected subcutaneously at a dose of 0.08mg/kg after 1.5 and 24 hours following CLP, respectively. Application of Co-Am antibiotics alone on the CLP model increased mortality. However, the combination of terazosin and Co-Am antibiotics has a protective effect on the model of CLP-induced sepsis. It was analyzed by the Kaplan-Merier test with the Log Rank algorithm. P value and the number of used mice are shown in the figure. T represents terazosin in the figure, and Co-Am represents a mixture of amoxicillin and clavulanate potassium at a weight ratio of 4:1.
Figure 10: shows a protein blot of the active form of caspase-3 in Raw 264.7 cells treated with LPS and IFNγ. The volumes of protein samples were the same among the three differently processed groups.
Figure 11: illustrates a scheme for chemical modification ofterazosin and Affigel immobilization.
Figure 12: Figure 12a shows that a significant protein band (between 43kd and 55kd, marked with arrows) appeared only in the experimental group obtained with terazosin agarose beads. The band was identified to be pgk1 with protein mass spectrometry. Figure b shows that a pgk1 band can be obtained from in vitro expressed pgk1 purified by terazosin agarose chromatography, indicating that terazosin can bind pgk1 directly.
Figure 13: shows the effects of different concentrations of terazosin on pgk1 activity.
Figure 14: shows that Raw 264.7 cells expressing pgk1 are resistant to apoptosis. Apoptotic cells are marked with Annexin V, which binds phosphatidylserine that becomes exposed on the cell membrane in cells undergoing apoptosis.
Figure 15: shows the protective effect after injection of lentivirus encoding pgk1 to mice having CLP-induced sepsis. Each mouse was injected 1 x 10⁷ unit of activity of the virus and tested a week later.
Figure 16: shows the blood sugar lowering effect of terazosin in mice.
Figure 17: shows the anti-thrombosis effect of terazosin.

### DETAILED DESCRIPTION

In a first aspect, the present invention relates to the compound of Formula I, or a pharmaceutical acceptable salt, solvate, ester, or prodrug thereof: 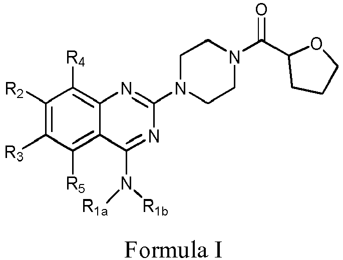 wherein,
R₁ₐ and R_{1b} are independently selected from H, NH₂, OH, C₁₋₆ alkyl, C₁₋₆ alkoxy-C₁₋₆ alkyl-, C₂₋₆ alkenyl, C₂₋₆ alkynyl, C₁₋₆ alkoxy, C₁₋₆ alkanoyl, aroyl, C₆₋₁₀ aryl, and C₅₋₆ cycloalkyl, or R₁ₐ and R_{1b} together with the nitrogen atom to which they are attached form a 5- or 6-membered ring, wherein said alkyl is optionally substituted with 1 to 3 substituents each independently selected from hydroxyl and a halogen;
R₂ and R₃ are independently selected from H, a halogen, C₁₋₆ alkyl, C₁₋₆ haloalkyl, C₂₋₆ alkenyl, C₂₋₆ alkynyl, CN, NO₂, NH₂, OH, C₁₋₆ alkoxy, C₁₋₆ alkoxy-C₁₋₆ alkoxy, C₁₋₆ alkanoyloxy, C₁₋₆ alkanoylamino, aroylamino, and saturated or unsaturated 5- or 6-membered carbocyclyl or heterocyclyl, C₁₋₆ alkanoyl, or R₂ and R₃ together with the ring atoms to which they are attached form a 5- or 6-membered carbocyclic or heterocyclic ring; and
R₄ and R₅ are independently selected from H, a halogen, CN, NO₂, NH₂, OH, C₁₋₆ alkyl, C₁₋₆ alkoxy-C₁₋₆ alkoxy, C₁₋₆ alkanoyloxy, C₁₋₆ haloalkyl, C₂₋₆ alkenyl, C₂₋₆ alkynyl, C₁₋₆ alkoxy, C₁₋₆ alkanoylamino, aroylamino, saturated or unsaturated 5- or 6-membered carbocyclyl or heterocyclyl, saturated or unsaturated 5- or 6-membered carbocyclyloxy or heterocyclyloxy, and C₁₋₆ alkanoyl.

In some embodiments of the present invention, R₁ₐ and R_{1b} are independently selected from H, NH₂, OH, C₁₋₆ alkyl, C₁₋₄ alkoxy -C₁₋₄ alkyl-, C₂₋₄ alkenyl, C₂₋₄ alkynyl, C₁₋₄ alkoxy, C₁₋₄ alkanoyl, benzoyl, phenyl, C₅₋₆ cycloalkyl, or R₁ₐ and R_{1b} together with the nitrogen atom to which they are attached form a 5- or 6-membered ring, wherein the alkyl is optionally substituted with 1 to 3 substituents each independently selected from hydroxyl and a halogen. In some embodiments, the R₁ₐ and R_{1b} are independently selected from H, -NH₂, -OH, CH₃C(O)-,-(CH₂)₂-O-(CH₂)₂-OH, -CH₂-CH=CH₂, -CH₂-C≡CH, -(CH₂)₅CH₃, -(CH₂)₄-CF₃, cyclohexyl,-CH₂-(CH₂)₃-CH₂-, -(CH₂)₂O(CH₂)₂-OH, -Ph, CH₃, -C(O)-CF₃, and -C(O)-Ph.

In some embodiments of the present invention, R₂ and R₃ are independently selected from H, a halogen, C₁₋₆ alkyl, C₁₋₆ haloalkyl, C₁₋₆ alkoxy, C₁₋₆ alkoxy-C₁₋₆ alkoxy, C₁₋₆ alkanoyloxy, C₁₋₆ alkanoylamino, aroylamino, and saturated or unsaturated 5- or 6-membered carbocyclyl or heterocyclyl, or R₂ and R₃ together with the ring atoms to which they are attached form a 5- or 6-membered carbocyclic or heterocyclic ring. In some embodiments, R₂ and R₃ are independently selected from H, CH₃O-, -CH₂-O-CH₂-, -O(CH₂)₂OC₂H₅, -OC(O)CH₃, -F, -CF₃, 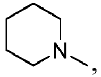 1,2-pyridine ring, -NHCOCH₃, -(CH₂)₂CH₃, -NHCOPh, and

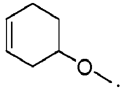

In some embodiments of the present invention, R₄ and R₅ are independently selected from H, a halogen, C₁₋₆ alkyl, C₁₋₆ alkoxy-C₁₋₆ alkoxy, C₁₋₆ alkanoyloxy, C₁₋₆ haloalkyl, C₁₋₆ alkoxy, C₁₋₆ alkanoylamino, aroylamino, saturated or unsaturated 5- or 6-membered carbocyclyl or heterocyclyl, and saturated or unsaturated 5- or 6-membered carbocyclyloxy or heterocyclyloxy. In some embodiments, R₄ and R₅ are independently selected from H, - O(CH₂)₂OC₂H₅, -OC(O)CH₃, -OCH₃, 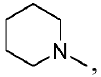 -CF₃, -F, -NHCOCH₃, -(CH₂)₃CH₃, -NHCOPh, and

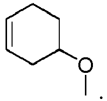

In some embodiments of the present invention, the alkyl, alkenyl and alkynyl groups are straight-chained or branched. In some embodiments, the C₁₋₆ alkyl group is selected from C₁₋₅ alkyl and C₁₋₄ alkyl, such as methyl, ethyl, n-propyl, isopropyl, n-butyl, sec-butyl, and t-butyl. In some embodiments, the C₂₋₆ alkenyl group is selected from C₂₋₅ alkenyl and C₂₋₄ alkenyl, such as vinyl, propenyl, and allyl. In some embodiments, the C₂₋₆alkynyl group is selected from C₂₋₅ alkynyl and C₂₋₄ alkynyl.

In some embodiments of the present invention, the C₅₋₆ cycloalkyl is selected from cyclopentyl and cyclohexyl.

In some embodiments of the present invention, the aryl is selected from phenyl and naphthyl, preferably phenyl.

In some embodiments of the present invention, the halogen is selected from -F,-Cl, -Br, and -I, preferably -F and -Cl.

In some embodiments of the present invention, the compound of Formula I is selected from the compounds numbered Co.1 to Co.33 (their structures are described in the preparation examples).

| **NO.** | **R₁ₐ** | **R_{1b}** | **R₂** | **R₃** | **R₄** | **R₅** | **Activity*** |
|---|---|---|---|---|---|---|---|
| Co.1 | CH₃C(O)- | H | CH₃O- | CH₃O- | H | H | 2.9 |
| Co.2 | -(CH₂)₂-O- (CH₂)₂-OH | H | CH₃O- | CH₃O- | H | H | 2.3 |
| Co.3 | -NH₂ | H | CH₃O- | CH₃O- | H | H | 1.9 |
| Co.4 | -OH | H | CH₃O- | CH₃O- | H | H | 3.4 |
| Co.5 | -CH₂-CH=CH₂ | H | CH₃O- | CH₃O- | H | H | 1.5 |
| Co.6 | -CH₂C≡CH | H | CH₃O- | CH₃O- | H | H | 1.2 |
| Co.7 | -(CH₂)₅CH₃ | H | CH₃O- | CH₃O- | H | H | 2.1 |
| Co.8 | -(CH₂)₄-CF₃ | H | CH₃O- | CH₃O- | H | H | 0.9 |
| Co.9 | Cyclohexyl | H | CH₃O- | CH₃O- | H | H | 1.4 |
| Co.10 | -CH₂-(CH₂)₃-CH₂- | | CH₃O- | CH₃O- | H | H | 0.7 |
| Co.11 | -(CH₂)₂-O- (CH₂)₂-OH | CH₃ | CH₃O- | CH₃O- | H | H | 2.1 |
| Co.12 | -Ph | H | CH₃O- | CH₃O- | H | H | 2.6 |
| Co.13 | -C(O)-CF₃ | H | CH₃O- | CH₃O- | H | H | 1.8 |
| Co.14 | -C(O)-Ph | H | CH₃O- | CH₃O- | H | H | 1.5 |
| Co.15 | H | H | -CH₂-O-CH₂- | | H | H | 0.6 |
| Co.16 | H | H | -O(CH₂)₂- O-C₂H₅ | -O(CH₂)₂- O-C₂H₅ | H | H | 1.7 |
| Co.17 | H | H | -OC(O)CH₃ | -OCH₃ | H | H | 1.1 |
| Co.18 | H | H | -F | -OCH₃ | H | H | 1.3 |
| Co.19 | H | H | 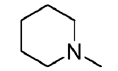 | -CF₃ | H | H | 1.4 |
| Co.20 | H | H | 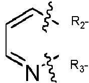 | | H | H | 1.9 |
| Co.21 | H | H | -NHCOCH₃ | -OCH₃ | H | H | 2.2 |
| Co.22 | H | H | -(CH₂)₂CH₃ | -OCH₃ | H | H | 2.4 |
| Co.23 | H | H | -NHCOPh | -OCH₃ | H | H | 0.7 |
| Co.24 | H | H | 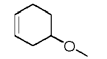 | -OCH₃ | H | H | 0.4 |
| Co.25 | H | H | -OCH₃ | -OCH₃ | -O(CH₂)₂- O-C₂H₅ | -O(CH₂)₂- O-C₂H₅ | 2.7 |
| Co.26 | H | H | -OCH₃ | -OCH₃ | -OC(O)CH₃ | -OCH₃ | 3.2 |
| Co.27 | H | H | -OCH₃ | -OCH₃ | 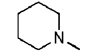 | -CF₃ | 0.9 |
| Co.28 | H | H | -OCH₃ | -OCH₃ | -F | -OCH₃ | 1.2 |
| Co.29 | H | H | -OCH₃ | -OCH₃ | -NHCOCH₃ | -OCH₃ | 2.6 |
| Co.30 | H | H | -OCH₃ | -OCH₃ | -(CH₂)₃CH₃ | -OCH₃ | 1.8 |
| Co.31 | H | H | -OCH₃ | -OCH₃ | -NHCOPh | -OCH₃ | 0.6 |
| Co.32 | H | H | -OCH₃ | -OCH₃ | 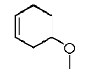 | -OCH₃ | 2.8 |
| Co.33 | H | H | CH₃O- | CH₃O- | H | H | 2.9 |

| | | | | | | | |
|---|---|---|---|---|---|---|---|
| Note: activity* indicates the times of pgk1 activity induced by 0.02µg/ml of the tested compound. | | | | | | | |

A second aspect of the present invention relates to the use of the compound of Formula I, such as terazosin, in manufacturing a medicament as an apoptosis inhibitor.

The second aspect of the present invention relates to the use of the compound of Formula I, such as terazosin, in manufacturing a medicament as a pgk1 activator.

The second aspect of the present invention relates to the use of the compound of Formula I, such as terazosin, in manufacturing a medicament for the treatment and/or prevention of sepsis and its complications.

In some embodiments of the present invention, the apoptosis inhibitor is used for the treatment and/or prevention of diseases and the diagnosis and/or detection thereof clinically or in a laboratory.

In some embodiments of the present invention, the pgk1 activator is used for the treatment and/or prevention of diseases and the diagnosis and/or detection thereof clinically or in a laboratory.

In some embodiments of the present invention, the sepsis is caused by bacteria and/or other microbial infections.

In some embodiments of the present invention, the complication of sepsis is selected from renal failure, respiratory failure, blood clotting disorders, organ damage (including but not limited to, toxic cardiomyopathy, encephalopathy, liver disease, and toxic intestinal paralysis, etc.), purulent meningitis, pneumonia, lung abscess, cellulitis, osteomyelitis, and pyelonephritis.

In some embodiments of the present invention, the compound of Formula I, for example terazosin, may be administered to a human or animal (e.g., a mammal) in a daily dose of 0.001∼5mg/kg, preferably 0.002∼4mg/kg, preferably 0.003∼3mg/kg, preferably 0.005∼2.5mg/kg, preferably 0.0075∼2mg/kg, preferably 0.01∼2mg/kg, preferably 0.01∼1mg/kg.

In some embodiments of the present invention, the compound of Formula I is terazosin.

In some embodiments of the present invention, the terazosin is pharmaceutically acceptable salt or solvate of Terazosin.

In some embodiments of the present invention, the terazosin is terazosin hydrochloride.

In some embodiments of the present invention, the terazosin is terazosin hydrochloride hydrate. In one embodiment, the terazosin is terazosin hydrochloride dihydrate.

In some embodiments of the present invention, wherein the medicament also further comprises at least one antimicrobial agent.

In some embodiments of the present invention, the medicament also further comprises at least one antimicrobial agent selected from penicillins, cephalosporins, β-lactamase inhibitors, aminoglycosides, tetracyclines, amphenicols antibiotics, macrolide antibiotics, sulfonamides, trimethoprim class, and quinolones.

In some embodiments of the present invention, the medicament also further comprises at least one antimicrobial agent selected from amoxicillin, penicillin, penicillinV, oxacillin, cloxacillin, flucloxacillin, ampicillin, piperacillin, azlocillin, potassium clavulanate, sulbactam, sultamicillin, tazobactam, aztreonam, and meropenem. In one embodiment, the medicament further comprises at least one antimicrobial drug selected from amoxicillin and clavulanate potassium. In one embodiment, the medicament further comprises amoxicillin and clavulanate potassium.

A third aspect of the present invention provides a pharmaceutical composition comprising a therapeutically and/or prophylactically effective amount of the compound of formula I, e.g., terazosin, or a pharmaceutically acceptable salt or solvate thereof, and optionally a pharmaceutically acceptable carrier.

In some embodiments of the present invention, the pharmaceutical composition further comprises at least one antimicrobial agent.

In some embodiments of the present invention, the at least one antimicrobial agent is selected from penicillins, cephalosporins, β-lactamase inhibitors, aminoglycosides, tetracyclines, amphenicols antibiotics, macrolide antibiotics, sulfonamides, trimethoprim class, and quinolones.

In some embodiments of the present invention, the at least one antimicrobial agent is selected from amoxicillin, penicillin, penicillinV, oxacillin, cloxacillin, flucloxacillin, ampicillin, piperacillin, azlocillin, potassium clavulanate, sulbactam, sultamicillin, tazobactam, aztreonam, and meropenem. In some embodiments, the at least one antimicrobial agent is selected from amoxicillin and clavulanate potassium. In one embodiment, the pharmaceutical composition further comprises amoxicillin and clavulanate potassium.

In some embodiments of the present invention, the pharmaceutical composition is used as an apoptosis inhibitor. In one embodiment, the apoptosis inhibitor is used for treatment and/or prevention of diseases and the diagnosis and/or detection thereof clinically or in a laboratory.

In some embodiments of the present invention, the pharmaceutical composition is used as a pgk1 activator. In one embodiment, the pgk1 activator is used for treatment and/or prevention of diseases and the diagnosis and/or detection thereof clinically or in a laboratory.

In some embodiments of the present invention, the pharmaceutical composition is used for treatment and/or prevention of sepsis and its complications. In one embodiment, the sepsis is caused by bacteria and/or other microbial infections. In one embodiment, the complications of sepsis are selected from renal failure, respiratory failure, blood clotting disorders, organ damage (including but not limited to, toxic cardiomyopathy, encephalopathy, liver disease, and toxic intestinal paralysis, etc.), purulent meningitis, pneumonia, lung abscess, cellulitis, osteomyelitis, and pyelonephritis.

In some embodiments of the present invention, the pharmaceutical composition is used for treatment and/or prevention of hyperglycemia or cerebral thrombosis, and their complications.

In some embodiments of the present invention, the compound of Formula I, for example, terazosin, is administered to a human or animal (e.g., a mammal) in a daily dose of 0.001∼5 mg/kg, preferably 0.002∼4mg/kg, preferably 0.003∼3mg/kg, preferably 0.005∼2.5mg/kg, preferably 0.0075∼2mg/kg, preferably 0.01∼2mg/kg, preferably 0.01∼1mg/kg.

A fourth aspect of the present invention provides a kit comprising a therapeutically and/or prophylactically effective amount of the compound of Formula I, e.g. terazosin, and/or a pharmaceutically acceptable salt or solvate thereof, and at least one antimicrobial agent.

In some embodiments of the present invention, the kit comprises the compound of Formula I, such as terazosin, or a pharmaceutically acceptable salt or solvate thereof, and the at least one antimicrobial agent in the same composition or in separate compositions.

In some embodiments of the present invention, the compound of Formula I, such as terazosin, or a pharmaceutically acceptable salt or solvate thereof, and the at least one antimicrobial drug are in separate compositions.

In some embodiments of the present invention, the kit comprises a first composition and a second composition separated from each other, wherein the first composition comprises a therapeutically and/or prophylactically effective amount of the compound of formula I, e.g. terazosin, or a pharmaceutically acceptable salt or solvate thereof, and optionally a pharmaceutically acceptable carrier, and the second composition comprises a therapeutically and/or prophylactically effective amount of an antimicrobial agent, and optionally a pharmaceutically acceptable carrier.

In some embodiments of the present invention, the antimicrobial agent is selected from Penicillins, cephalosporins, β-lactamase inhibitors, aminoglycosides, tetracyclines, amphenicols antibiotics, macrolide antibiotics, sulfonamides, trimethoprim class, and quinolones.

In some embodiments of the present invention, the antimicrobial agent is selected from amoxicillin, penicillin, penicillinV, oxacillin, cloxacillin, flucloxacillin, ampicillin, piperacillin, azlocillin, potassium clavulanate, sulbactam, sultamicillin, tazobactam, aztreonam, and meropenem. In one embodiment, the pharmaceutical composition further comprises at least one antimicrobial agent selected from amoxicillin and clavulanate potassium. In one embodiment, the antimicrobial agent comprises amoxicillin and clavulanate potassium.

In some embodiments of the present invention, the kit is used as an apoptosis inhibitor. In one embodiment, the apoptosis inhibitor is used for treatment and/or prevention of diseases and the diagnosis and/or detection thereof clinically or in a laboratory.

In some embodiments of the present invention, the kit is used as a pgk1 activator. In one embodiment, the pgk1 activator is used for treatment and/or prevention of diseases and the diagnosis and/or detection thereof clinically or in a laboratory.

In some embodiments of the present invention, the kit is used for treatment and/or prevention of sepsis and its complications. In some embodiments, the sepsis is caused by bacteria and/or other microbial infections. In some embodiments, the complications of sepsis are selected from renal failure, respiratory failure, blood clotting disorders, organ damage (including but not limited to, toxic cardiomyopathy, encephalopathy, liver disease, and toxic intestinal paralysis, etc.), purulent meningitis, pneumonia, lung abscess, cellulitis, osteomyelitis, and pyelonephritis.

In some embodiments of the present invention, the kit is used for treatment and/or prevention of hyperglycemia or cerebral thrombosis, and their complications.

In some embodiments of the present invention, the compound of Formula I, for example, terazosin, is administered to a human or animal (e.g., a mammal) in a daily dose of 0.001∼5 mg/kg, preferably 0.002∼4mg/kg, preferably 0.003∼3mg/kg, preferably 0.005∼2.5mg/kg, preferably 0.0075∼2mg/kg, preferably 0.01∼2mg/kg, preferably 0.01∼1mg/kg.

A fifth aspect of the present invention relates to a method of inhibiting apoptosis in a subject in need thereof or in a biological sample. The method comprises administering to the subject in need thereof or the biological sample an effective amount of the compound of formula I, for example, terazosin,

The fifth aspect of the present invention also relates to a method of activating pgk1 in a subject in need thereof or in a biological sample. The method comprises administering to the subject in need thereof or the biological sample an effective amount of the compound of formula I, e.g. terazosin.

The fifth aspect of the present invention also relates to a method for treatment and/or prevention of sepsis and its complications in a subject in need thereof. The method comprises administering to the subject in need thereof an effective amount of the compound of formula I, e.g. terazosin.

The fifth aspect of the present invention also relates to a method for treatment and/or prevention of hyperglycemia or cerebral disease, and its complications in a subject in need thereof. The method comprises administering to the subject in need an effective amount of the compound of formula I, e.g. terazosin.

In some embodiments of the present invention, the sepsis is caused by bacteria and/or other microbial infections.

In some embodiments of the present invention, the complication of sepsis is selected from renal failure, respiratory failure, blood clotting disorders, organ damage (including but not limited to, toxic cardiomyopathy, encephalopathy, liver disease, and toxic intestinal paralysis, etc.), purulent meningitis , pneumonia, lung abscess, cellulitis, osteomyelitis, and pyelonephritis.

In some embodiments of the present invention, the compound of Formula I, for example, terazosin, is administered to a human or animal (e.g., a mammal) in a daily dose of 0.001∼5 mg/kg, preferably 0.002∼4mg/kg, preferably 0.003∼3mg/kg, preferably 0.005∼2.5mg/kg, preferably 0.0075∼2mg/kg, preferably 0.01∼2mg/kg, preferably 0.01∼1mg/kg.

In some embodiments of the present invention, the compound of Formula I is terazosin.

In some embodiments of the present invention, the terazosin is a pharmaceutically acceptable salt or solvate of terazosin.

In some embodiments of the present invention, the terazosin is terazosin hydrochloride.

In some embodiments of the present invention, the terazosin is terazosin hydrochloride hydrate. In one embodiment, the terazosin is terazosin hydrochloride dihydrate.

In some embodiments of the present invention, the method further comprises administering to the subject or biological sample an effective amount of at least one antimicrobial agent.

In some embodiments of the present invention, the antimicrobial agent is selected from penicillins, cephalosporins, β-lactamase inhibitors, aminoglycosides, tetracyclines, amphenicols antibiotics, macrolide antibiotics, sulfonamides, trimethoprim class, and quinolones.

In some embodiments of the present invention, the antimicrobial agent is selected from amoxicillin, penicillin, penicillinV, oxacillin, cloxacillin, flucloxacillin, ampicillin, piperacillin, azlocillin, potassium clavulanate, sulbactam, sultamicillin, tazobactam, aztreonam, and meropenem. In some embodiments, the at least one antimicrobial drug may be selected from amoxicillin and clavulanate potassium. In some embodiments, the method may further comprise administering to the subject or biological sample amoxicillin and clavulanate potassium.

A sixth aspect of the present disclosure provides the compound of formula I, e.g. terazosin, for use as an apoptosis inhibitor.

The sixth aspect of the present disclosure also provides the compound of formula I, e.g., terazosin, for use as a pgk1 activator.

The sixth aspect of the present disclosure also provides the compound of formula I, e.g., terazosin, for the treatment and/or prevention of sepsis and its complications.

The sixth aspect of the present disclosure also provides the compound of formula I, e.g., terazosin, for the treatment and/or prevention of hyperglycemia or cerebral disease, and its complications.

In some embodiments of the present invention, the apoptosis inhibitor may be suitable for the treatment and/or prevention of diseases and the diagnosis and/or detection thereof clinically or in a laboratory.

In some embodiments of the present invention, the pgk1 activator may be suitable for the treatment and/or prevention of diseases and the diagnosis and/or detection thereof clinically or in a laboratory.

In some embodiments of the present invention, the sepsis may be caused by bacteria and/or other microbial infections.

In some embodiments of the present invention, the sepsis may include at least one complication selected from renal failure, respiratory failure, blood clotting disorders, organ damage (including but not limited to, toxic cardiomyopathy, encephalopathy, liver disease, and toxic intestinal paralysis, etc.), purulent meningitis, pneumonia, lung abscess, cellulitis, osteomyelitis, and pyelonephritis.

In some embodiments of the present invention, wherein the compound of Formula I may be administered to a human or animal (e.g., a mammal) in a daily dose ranging from 0.001mg/kg to 5mg/kg, such as, e.g., from 0.002mg/kg to 4mg/kg, from 0.003mg/kg to 3mg/kg, from 0.005mg/kg to 2.5mg/kg, from 0.0075mg/kg to 2mg/kg, from 0.01mg/kg to 2mg/kg, or from 0.01mg/kg to 1mg/kg.

In some embodiments of the present invention, the compound of Formula I is terazosin.

In some embodiments of the present invention, the terazosin is pharmaceutically acceptable salt or solvate of terazosin.

In some embodiments of the present invention, the terazosin is terazosin hydrochloride.

In some embodiments of the present invention, the terazosin is terazosin hydrochloride hydrate, e.g., terazosin hydrochloride dihydrate.

In some embodiments of the present invention, the compound of Formula I may be in a combination with at least one antimicrobial agent.

In some embodiments of the present invention, the at least one antimicrobial agent is selected from penicillins, cephalosporins, β-lactamase inhibitors, aminoglycosides, tetracyclines, amphenicols antibiotics, macrolide antibiotics, sulfonamides, trimethoprim class, and quinolones.

In some embodiments of the present invention, the at least one antimicrobial agent is selected from amoxicillin, penicillin, penicillinV, oxacillin, cloxacillin, flucloxacillin, ampicillin, piperacillin, azlocillin, potassium clavulanate, sulbactam, sultamicillin, tazobactam, aztreonam, and meropenem. In some embodiments, the at least one antimicrobial agent is selected from amoxicillin and clavulanate potassium. In one embodiment, the antimicrobial agent comprises amoxicillin and clavulanate potassium.

In any aspect of the present invention, the amount of the compound of formula I, e.g. terazosin, can be selected by referring to conventional clinical dosages. For example, for treatment and/or prevention of sepsis and its complications in a human, the dosage of terazosin may be 0.01∼100 times in clinical, preferably 0.02∼80 times, preferably 0.05∼20 times, preferably 0.1∼10 times, preferably 0.1∼5 times, preferably 0.2∼5 times, preferably 0.2∼2 times, of the dosage of terazosin currently selected for clinical treatment of other diseases (e.g., hypertension).

In any aspect of the present invention, the amount of antimicrobial agent can be selected by referring to conventional clinical dosages. For example, for treatment and/or prevention of sepsis and its complications in a human, the dosage of the antimicrobial agent may be 0.01∼100 times in the clinical, preferably 0.02∼80 times, preferably 0.05∼20 times, preferably 0.1∼10 times, preferably 0.2∼5 times, of the dosage of the antimicrobial drug currently selected for clinical treatment of other diseases (e.g., infections).

In any aspect of the present invention, the amount of amoxicillin and/or clavulanate potassium can be selected by referring to conventional clinical dosages. For example, for treatment and/or prevention of sepsis and its complications in a human, the dosage of amoxicillin and/or clavulanate potassium may be 0.01∼100 times, preferably 0.02∼80 times, preferably 0.05∼20 times, preferably 0.1∼10 times, preferably 0.2∼5 times, of the dosage of amoxicillin and/or clavulanate potassium currently selected for clinical treatment of other diseases (e.g., infections).

In some embodiments of any aspect of the present invention, the compound of Formula I is a compound other than the one numbered Co.33.

The feature in any aspect or any embodiment of any aspect of the present invention is equally applicable to any other aspect or any other embodiment of any aspect of the present invention, as long as they do not contradict each other. When applicable to each other, if necessary, the corresponding features can be appropriately modified.

The various aspects and features of the present invention will be further described below.

The entire contents of all the references cited in the present invention are incorporated herein by reference, and if the disclosure in these documents is inconsistent with the present invention, the present invention shall prevail. In addition, the present invention uses various terms and phrases that have a general meaning known by those skilled in the art. Even so, the present invention still contains more detailed illustration and explanation of these terms and phrases. If there is any inconsistency between the meanings of the terms and phrases as disclosed herein and those known in the art, the present invention shall prevail.

As used herein, the term "septicemia," also called "sepsis", has the meaning as known in the art, and refers to a kind of serious systemic infection caused by the growth of and the toxins produced by bacteria and/or other microorganisms that have entered the blood circulation. Clinical manifestations are fever, severe toxemia, rash petechiae, hepatosplenomegaly, and increased white blood cell count and so on. Gram-positive cocci caused sepsis may contain migratory lesions; Gram-negative bacilli caused sepsis may contain combined septic shock. Sepsis may also be accompanied with multiple abscesses.

As used herein, the term "pgk1 activator" refers to activator of phosphoglycerate kinase 1 (abbreviated as pgk1), which can activate pgk1, and thus can be understood by those skilled in the art to be useful for the treatment, prevention, mitigation, and/or relief of a disease or disorder.

As used herein, the term "subject" or "patient" may refer to animals receiving the compositions described herein to treat, prevent, mitigate, and/or relieve a disease, condition, or symptom, including mammals, such as, e.g., humans, dogs, monkeys, cows, horses and so on.

As used herein, the term "disease or condition" refers to a physical condition of the subject, which is related to the diseases or symptoms described herein.

As used herein, "%", unless otherwise specified, refers to weight/weight percentage if the total material is solid, or weight/volume percentage if the total material is liquid. If the total material is liquid and the solute is liquid, the percentage of the solute refers to volume/volume percentage.

Terazosin ((4-(4-amino-6,7-dimethoxyquinazolin-2-yl)piperazin-1-yl)(tetrahydrofuran-2-yl)methanone, C₁₉H₂₅N₅O₄) has the following chemical structural formula:

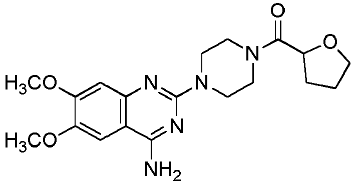

As used herein, terazosin also refers to pharmaceutically acceptable salts (e.g. hydrochloride) of the compound having the above formula, and or solvates (such as, e.g., hydrates) of the compound having the above formula and its salt. In one preferred embodiment of the present invention, terazosin refers to terazosin hydrochloride dihydrate. In the present invention, terazosin was used as a typical example of the compounds of formula I to conduct extensive research to demonstrate the unexpected effect; in particular, the test biological tests hereinafter, unless otherwise indicated, the terazosin used is terazosin hydrochloride dehydrate.

The present invention attempts to screening apoptosis inhibitors from clinical medicines. First, analysis of gene expression was conducted via microarray technology using a Drosophila model of apoptosis. Then, apoptosis inhibitor candidates were determined using a connectivity map (Cmap) through bioinformatic analysis. Next, drug candidates that inhibit apoptosis in Drosophila were identified. Accordingly, terazosin was selected, which has been used clinically for relieving hypertension. Furthermore, it was discovered that terazosin can inhibit apoptosis of macrophages induced by bacterial endotoxin (lipopolysaccharide, LPS, 2µg/ml) and interferon γ (IFNγ, 50U/ml). In addition, it was also found that, in the three experimental models of sepsis, terazosin can notably reduce the mortality rate of mice. Interestingly, compared to use of antibiotics alone, combination of terazosin and antibiotics can produce better protection. The results show that terazosin is a novel apoptosis inhibitor, which can be used in combination with antibiotic therapy.

Drosophila is an important animal model system for drug screening. In addition, the caspase-mediated apoptotic pathway is highly conserved between Diptera and humans. For example, Reaper (rpr) plays a major role in Drosophila apoptosis (White *et al.,* 1994). Heat-induced rpr expression causes widespread, abnormal apoptosis and organ death (White *et al.,* 1996). The present invention investigated whether a Drosophila model of apoptosis can be used to screen for apoptosis inhibitors.

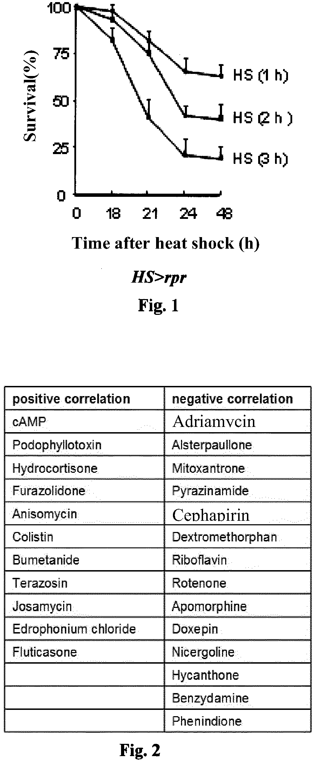

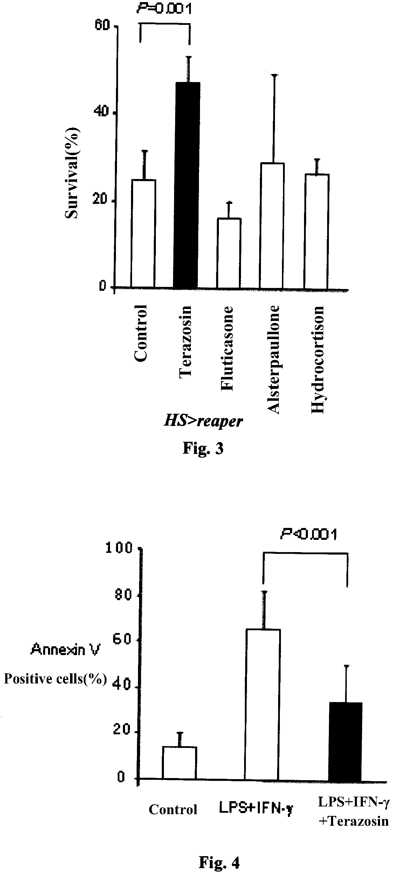

HS-Gal4 (a promoter that can be expressed in whole body and activated by heat), can promote expression of any genes having repeated UAS (upstream activating sequence) at the 5' region. The HS-Gal4 and UAS-Reaper Drosophila were crossed at 18 °C to produce hybrids referred to as HS> rpr, and the female offspring underwent normal development. However, with heat shock for 2-3 hours at 37 °C, about 50-70% of the offspring died after 14-24 h (Fig. 1). 25 compounds were selected (Fig. 2) to determine their impact on the lethal rate of HS> rpr. It was found that only terazosin can significantly improve the survival rate of HS>rpr Drosophila (Fig. 3).

To test whether terazosin can also inhibit apoptosis in cultured mammalian cells, apoptosis was induced in RAW264.7 macrophage cells by LPS and IFNγ that are known inducing agents. Cells were stained with Annexin V to examine apoptosis (Fig. 4). The present inventors have found that LPS (2 µg/ml) and IFNγ (50 U/ml) treatment caused cell membrane damaged and eversion, and thus staining by Annexin V. This is a classic pattern of apoptosis. Terazosin (4 µg/ml) can reduced 50% of apoptosis (Fig. 4). It is surprisingly found that terazosin can effectively inhibit apoptosis and be useful for the treatment and/or prevention of sepsis and its complications.

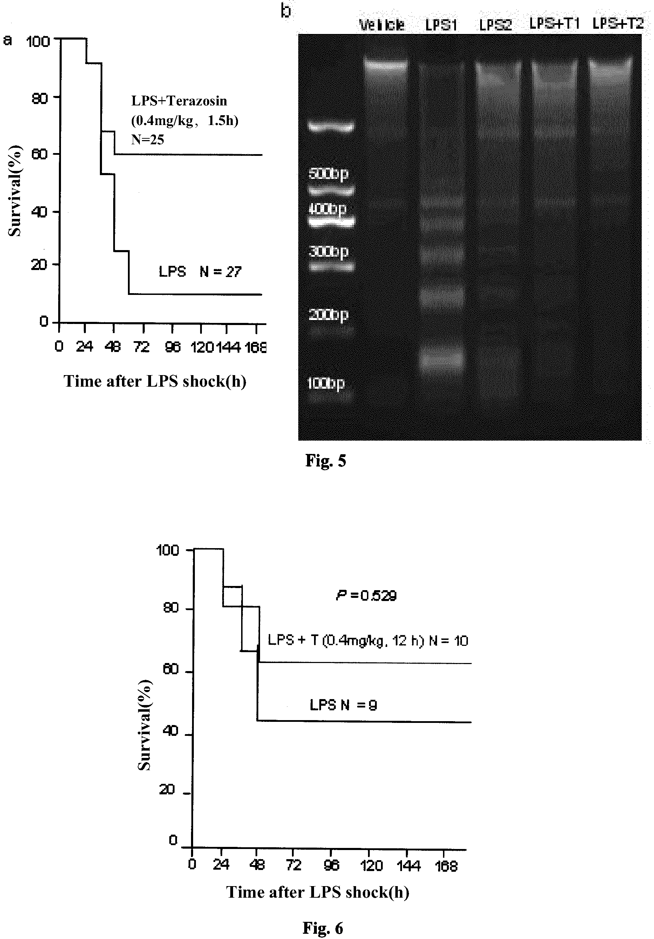

To further investigate the effect of terazosin in mammalian models of sepsis, LPS treated mice were studied. The present inventors have found that, administration of terazosin (0.4 mg/kg i.p.) at 1.5 hours after LPS injection (13.5 mg/kg, i.p.) significantly increased the survival rate of mice (Fig. 5a). To confirm the effect of terazosin on apoptosis, apoptotic markers of DNA breaks were examined. As an organ that produces immune cells, thymus during septicemia are sensitive to apoptosis (Ayala *et al.,* 1998). Thus, genomic DNAs of the thymus from mice treated with LPS and mice treated with LPS and terazosin were compared. The results show that notable DNA ladders occurred in the mice treated with LPS, but were significantly reduced in mice treated with terazosin (Fig. 5b). To test whether terazosin may play a role in later phases of septicemia, terazosin was injected at 12 hours after LPS treatment. Terazosin still showed satisfactory results (Fig. 6).

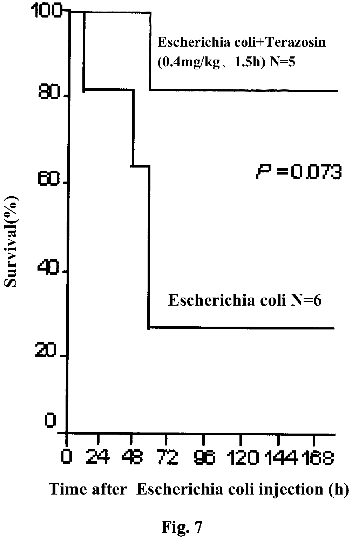

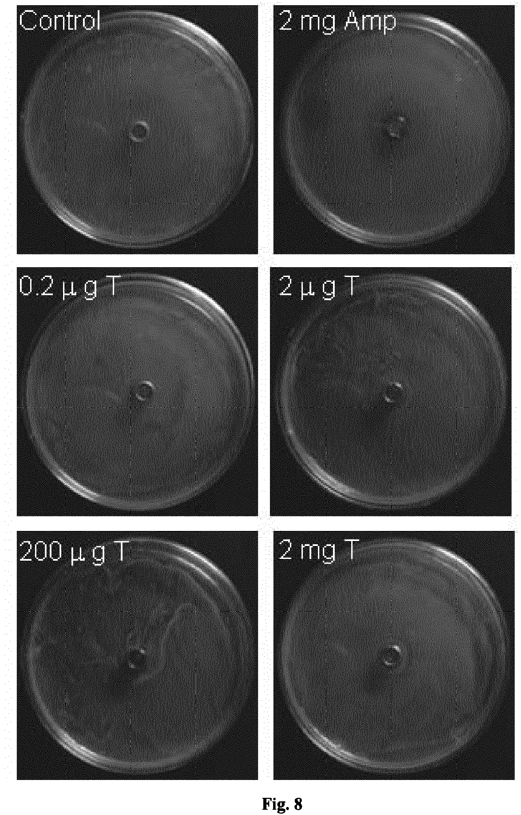

Terazosin was tested in a model of septicemia caused by E. coli injected. The results show that terazosin can protect mice from E. coli-induced death (Fig. 7). To determine the potential antibacterial effect, the growth of E. coli was examined in the presence of terazosin. The results show that, as opposed to ampicillin, terazosin cannot inhibit the growth of E. coli (FIG. 8).

Because E. coli and LPS models can only simulate Gram-negative bacteria infections, terazosin was further tested in a cecal ligation and puncture (CLP) model, which is believed to be the classic standard model of experimental septicemia (Parker and Watkins, 2001; Wichterman et al., 1980). First, 0.4 mg/kg of terazosin (same concentration as used in the LPS model) was tested. However, in the first 5 days, mice injected with terazosin died faster than the control group (data not shown). This may be due to severe cardiac dysfunction and hypotension induced by CLP. Thus, the blood pressure lowering function of the drug may have masked its anti-apoptotic effect. To solve this problem, a reduced concentration of terazosin (0.08 mg/kg) was administered, and the blood pressure of the injected mice was found completely normal. This result is consistent with the report that terazosin does not decrease blood pressure in rats (Kyncl et al., 1985). Interestingly, injected respectively at 1.5 hours and 24 hours after surgery, terazosin significantly promoted the survival of mice in the CLP model (Fig. 9). In addition, the combination of terazosin and antibiotics was tested. Terazosin was combined with amoxicillin and clavulanate potassium (Co-Am, amoxicillin and clavulanate potassium in a weight ratio of 4: 1, by way of a specific example elsewhere or instance mentioned in the present invention, also means a 4 : 1 ratio. Skilled in the art can understand, when a combination of amoxicillin and clavulanate potassium is referred to, the weight ratio of amoxicillin and clavulanate potassium can be from 1:1 to 10:1, especially 1:1-7:1, for example, about 1:1, about 4:1, about 7:1). The present inventors have found that, the combination of terazosin and Co-Am showed a stronger protective effect than Co-Am alone (Fig. 9). Furthermore, the combination had a therapeutic effect when administered 6 hours after CLP.

To test whether the protective effect of terazosin is related to its function as an α1-adrenergic receptor inhibitor, Brad prazosin (0.4 mg/kg, i.p.), another α1-prostaglandin inhibitor (Cavero et al., 1977), was tested. It was shown that Brad prazosin did not have any effect on sepsis caused by LPS. These results indicate that the activity of terazosin significantly increased the survival rate of LPS-induced sepsis may be due to its function as an inhibitor of apoptosis rather than that of α1-adrenoceptor.

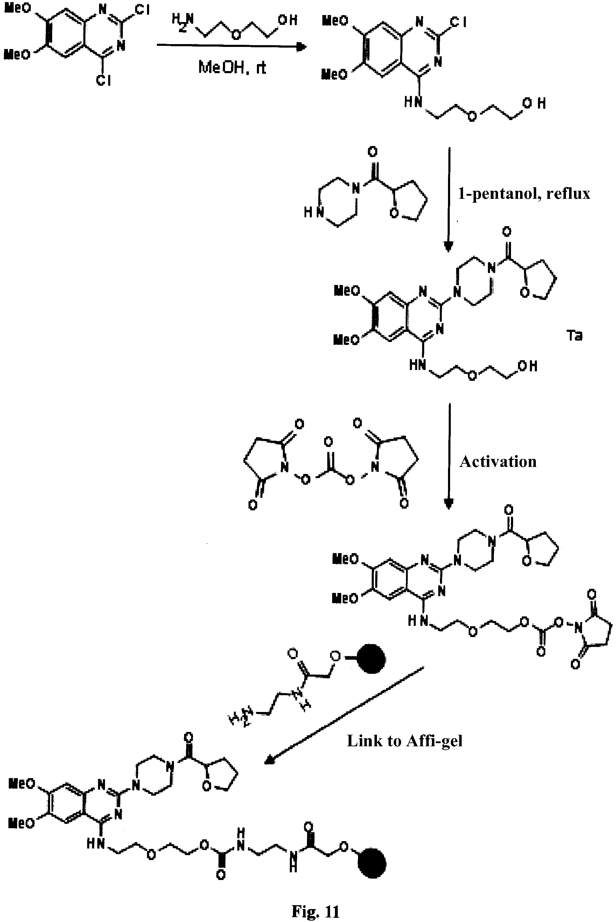

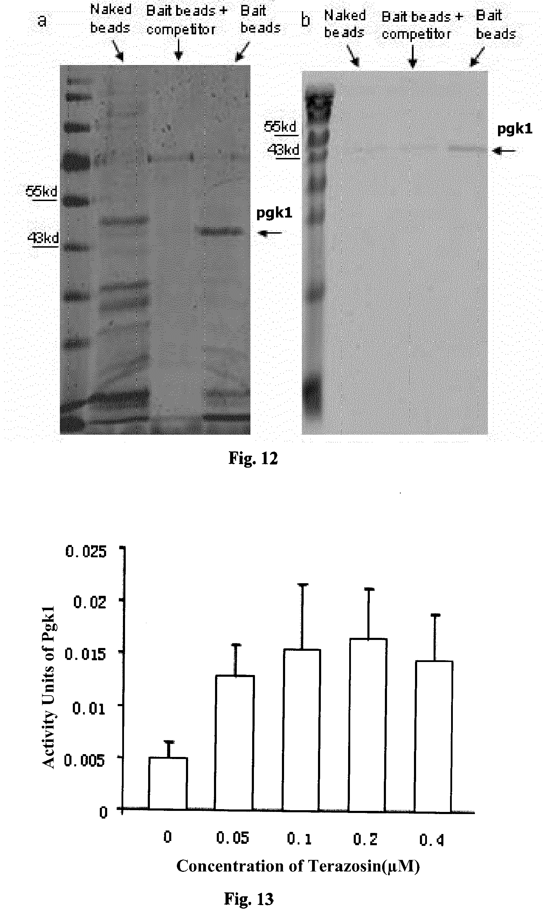

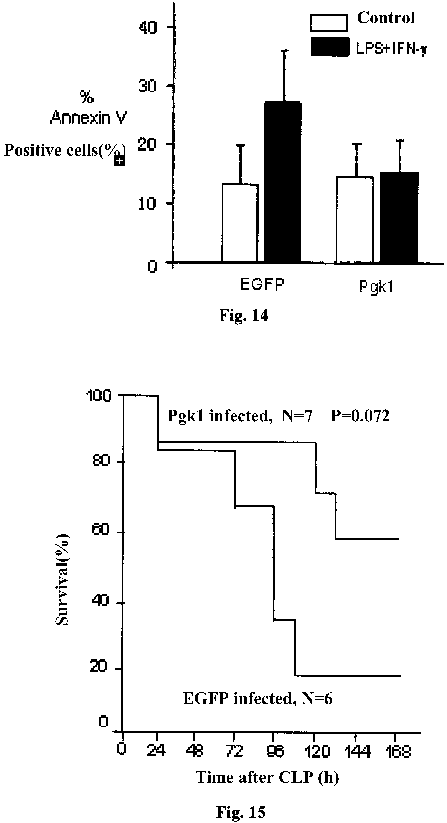

To study the biological mechanism of apoptosis inhibition by terazosin, the potential effect of terazosin in inhibiting apoptosis protease was examined. Apoptosis in Drosophila is realized mainly through intrinsic caspase activation (McCall and Steller, 1997). However, Western blot analysis showed that the active form of caspase-3 (homologous gene of Dpc-1 and drICE in Drosophila) was decreased by terazosin (Fig. 10). Therefore, terazosin may be able to adjust targets upstream of caspase-3. To identify the target genes, terazosin was immobilized to agarose beads, using a synthetic scheme as shown in Fig. 11. Proteins were captured by the beads from Raw 264.7 cell lysate, and an extra band was found by comparing the blank control and the drug competition group (Fig. 12a). This band was identified to be pgk1 by mass spectrometry. In order to determine whether terazosin can directly bind to pgk1, mouse pgk1 protein was expressed and purified from bacteria, and it was found that terazosin can directly bind pgk1 in vitro (Fig. 12b). Because overexpression of pgk1 in yeast can inhibit apoptosis (Mazzoni et al., 2009), the present inventors have speculated terazosin may be able to activate pgk1. To prove this hypothesis, the activity of pgk1 was examined in vitro. It was found that 0.05µM terazosin can increase the enzymatic activity of pgk1 to about 3 times, while 0.4µM terazosin reduced the extent to which the enzymatic activity of pgk1 can be increased (Fig. 13). If the enzymatic activity of pgk1 is anti-apoptotic, overexpression Pkg1 should inhibit apoptosis. The results show that LPS and IFNγ induced apoptosis was significantly reduced in RAW264.7 cell lines established by lentivirus expressing pgk1, whereas no change of apoptosis occurred with overexpression of EGFP (Fig. 14 ). Furthermore, mice were subcutaneously injected with lentivirus expressing EGFP (control) or pgk1 . CLP was done one week after the injection. It was found that, compared with mice transfected with GFP, mice injected with pgk1-expressing virus showed a significantly increased survival rate (Fig. 15). Without being bound by any particular theory, the present inventors believe pgk1 is a new target for terazosin.

Studies in the present invention showed that terazosin can be easily applied clinically, without modifying the existing antibiotic treatment procedures. The biological experiments of the present invention will be described in detail as follows.

### Examples

### A. Preparation of compounds

In the following Preparation Examples, some embodiments of the compounds of Formula I of the present invention were exemplarily prepared, including exemplary compounds represented by Co.1 to Co.32, and Co.33 represents terazosin.

### Example 1. Preparation of Co.1

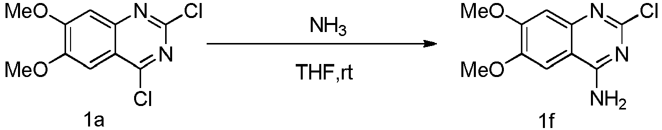

Step 1: Ammonia gas was introduced into the solution of compound 1a (50mmol) in 200mL tetrahydrofuran, the reaction was carried out at 25 °C for 36 hrs. A large amount of white solid precipitated and was filtered, and the resulting white solid was washed with tetrahydrofuran to give the final product 1f. Yield: 63%.

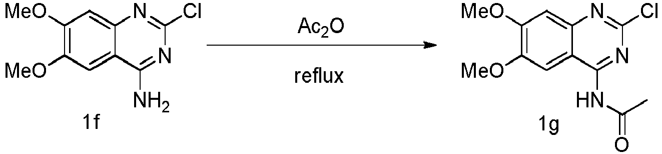

Step 2: Compound 1f (10mmol) was dissolved in 15mL acetic anhydride, the reaction mixture was refluxed for 2 hrs, cooled to room temperature, a lot of white solid precipitated and was filtered, and the resulting white solid was washed with tetrahydrofuran to give the final product 1g. Yield: 63%.

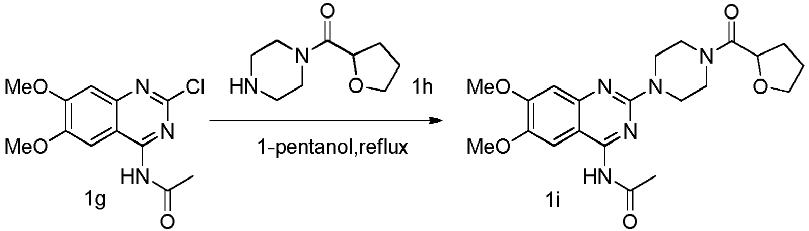

Step 3: Under argon atmosphere, compound 1h (2mmol) was added to the solution of compound 1g (2mmol) in 1-pentanol. After the reaction mixture was refluxed for 4.5 hrs, then placed at 0-5 °C for crystallizing on standing. The resulting white crystals were washed twice with 10mL acetone, recrystallized with diethyl ether/methanol to give the final product of the compound Co.1. Yield: 60%.

1H NMR (300 MHz, CDCl₃): 1.84 (m, 2H, thf-H), 2.03 (m, 2H, thf-H), d 2.61 (s, 3H, CH₃), 3.47-4.07 (m, 11H, thf-H, pip-H and CH₂CH₂O), 3.93 (s, 3H, OCH₃), 3.97 (s, 3H, OCH₃), 4.82 (m, 1H, thf-2H), 7.16 (s, 1H, Ar-5H), 7.72 (s, 1H, Ar-8H); HR-MS (ESI-positive): 430.20928 (M+H) (calculated: 430.20904); Elemental analysis: (C: 58.74; H: 6.34; N: 16.30; O: 18.63); calculated: (C: 58.73; H: 6.34; N: 16.31; O: 18.63).

### Example 2. Preparation of Co.2

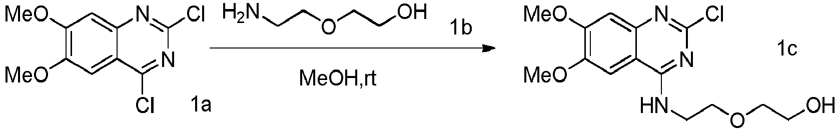

Step 1: Compound 1b (20 mmol) was added into the solution of compound 1a (20 mmol) in 100mL methanol, the reaction was carried out at 25 °C for 4 hrs. Thin layer chromatography (TLC) indicated compound 1a was completely converted, 100 mL of diethyl ether was added, then the mixture was placed at -20 °C for crystallizing on standing. The resulting white solid was recrystallized with petroleum ether/ethyl acetate to obtain the final product 1c. Yield: 41%.

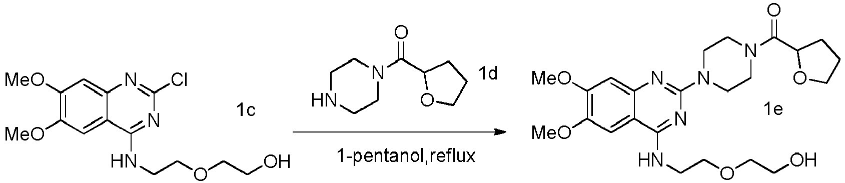

Step 2: Under argon atmosphere, compound 1d (2 mmol) was added into the solution of compound 1c (2mmol) in 1-pentanol. After the reaction mixture was refluxed for 4.5 hrs, then placed at 0-5 °C for crystallizing on standing. The resulting white crystals were washed twice with 10mL acetone and recrystallized with diethyl ether/methanol to give the final product of the compound 1e, Co.2. Yield: 62%.

1H NMR (300 MHz, DMSO-d6): 1.84 (m, 2H, thf-H), 2.03 (m, 2H, thf-H), 3.47-4.07 (m, 14H, thf-H, pip-H and CH₂CH₂O), 3.84 (s, 3H, OCH₃), 3.87 (s, 3H, OCH₃) 4.73 (m, 1H, thf-2H), 7.16 (s, 1H, Ar-5H), 7.72 (s, 1H, Ar-8H);13 C NMR (DMSO-d6): d 25.2, 27.9, 40.9, 44.1, 44.6, 55.8,56.2, 60.1,68.1, 68.2, 72.1, 74.9, 102.4, 104.4, 106.9, 146.2, 154.4, 154.2, 158.6, 169.6; HR-MS (ESI-positive) : 476.25120 (M+H) (Calculated: 476.25091); Elementary analysis : C, 58.08; H, 7.00; N, 14.73; O, 20.19; Calculated: (C, 58.09 ; H, 6.99 ; N, 14.73 ; 0, 20.19).

### Example 3. Preparation of Co.3

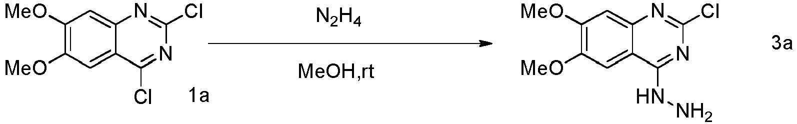

Step 1: Hydrazine hydrate (20 mmol) was added into the solution of compound 1a (20 mmol) in 100 mL methanol, the reaction was carried out at 25 °C for 4 hrs. TLC indicated compound 1a was completely converted, 100 mL of diethyl ether was added, mixed and placed at -20 °C for crystallizing on standing. The resulting white solid was recrystallized with petroleum ether/ethyl acetate to give the final product 3a. Yield: 61%.

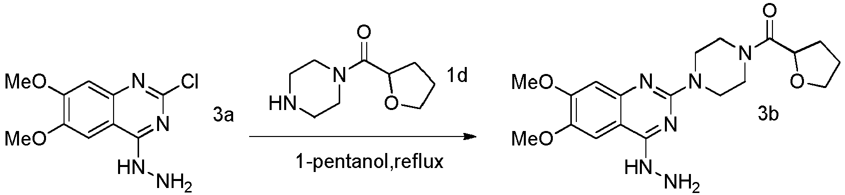

Step 2: Under argon atmosphere, compound 1d (2 mmol) was added to the solution of compound 3a (2 mmol) in 1-pentanol. The reaction mixture was refluxed for 4.5 hrs, placed at 0-5 °C for crystallizing on standing. The resulting white crystals were washed twice with 10mL acetone, and recrystallized with diethyl ether/methanol to give the final product of the compound 3b, Co.3. Yield: 31%.

1H NMR (300 MHz, DMSO-d6) : d 1.87 (m, 2H, thf-H), 2.03 (m, 2H, thf-H), 3.43-4.07 (m, 6H, thf-H, pip-H), 3.86 (s, 3H, OCH₃), 3.88 (s, 3H, OCH₃), 4.63 (m, 1H, thf-2H), 7.16 (s, 1H, Ar-5H), 7.82 (s, 1H, Ar-8H); HR-MS (ESI-positive) : 403.20938 (M+H) (Calculated : 403.20946).

### Example 4. Preparation of Co.4

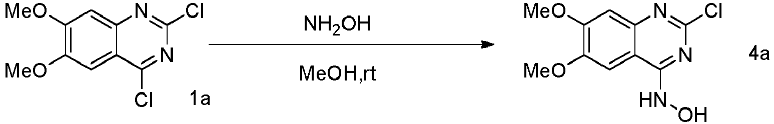

Step 1: Hydroxylamine hydrate (20 mmol) was added into the solution of compound 1a (20 mmol) in 100 mL methanol, the reaction was carried out at 25 °C for 3 hrs. TLC indicated compound 1a was completely converted, 100 mL of diethyl ether was added, mixed and placed at -20 °C for crystallizing on standing. The resulting white solid was recrystallized with petroleum ether/ethyl acetate to give the final product 4a. Yield: 88%.

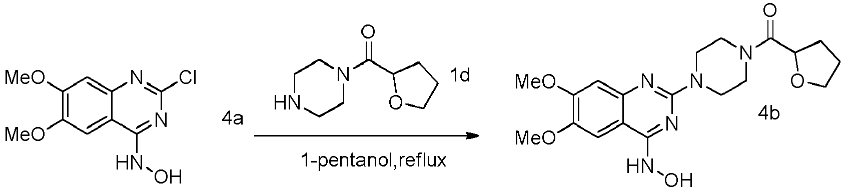

Step 2: Under argon atmosphere, compound 1d (2 mmol) was added to the solution of compound 4a (2 mmol) in 1-pentanol. The reaction mixture was refluxed for 4.5 hrs, placed at 0-5 °C for crystallizing on standing. The resulting white crystals were washed twice with 10mL acetone, and recrystallized with diethyl ether/methanol to give the final product of the compound 4b, Co.4. Yield: 75%.

1H NMR (300 MHz, DMSO-d6): 1.81 (m, 2H, thf-H), 2.03 (m, 2H, thf-H), 3.47-4.07 (m, 6H, thf-H, pip-H), 3.87 (s, 3H, OCH₃), 3.94 (s, 3H, OCH₃), 4.43 (m, 1H, thf-2H), 7.26 (s, 1H, Ar-5H), 7.74 (s, 1H, Ar-8H); HR-MS (ESI-positive) : 404.19339 (M+H) (Calculated: 404.19341).

### Example 5. Preparation of Co.5

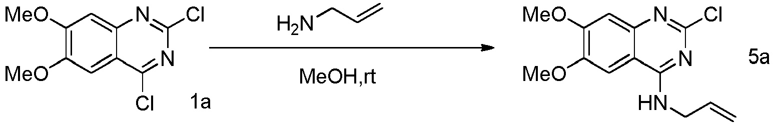

Step 1: Allylamine (22 mmol) was added into the solution of compound 1a (20 mmol) in 100 mL methanol, the reaction was carried out at 25 °C for 8 hrs. TLC indicated compound 1a was completely converted, 100 mL of diethyl ether was added, mixed and placed at -20 °C for crystallizing on standing. The resulting white solid was recrystallized with petroleum ether/ethyl acetate to give the final product 5a. Yield: 31 %.

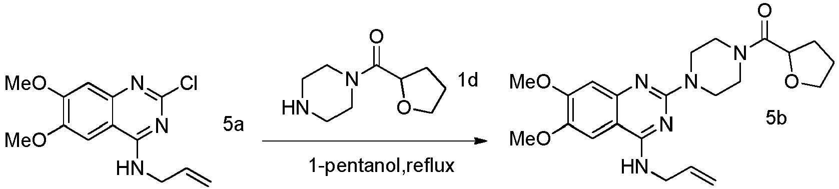

Step 2: Under argon atmosphere, compound 1d (2 mmol) was added to the solution of compound 4a (2 mmol) in 1-pentanol. The reaction mixture was refluxed for 4.5 hrs, placed at 0-5 °C for crystallizing on standing. The resulting white crystals were washed twice with 10mL acetone, and recrystallized with diethyl ether/methanol to give the final product of the compound 5b, Co.5. Yield: 75%.

1H NMR (300 MHz, DMSO-d6): 1.82 (m, 2H, thf-H), 2.03 (m, 2H, thf-H), 3.47-4.07 (m, 6H, thf-H, pip-H), 3.87 (s, 3H, OCH₃), 3.94 (s, 3H, OCH₃), 4.04 (d, 2H), 4.43 (m, 1H, thf-2H), 5.19-5.23 (m, 2H), 5.82-5.88 (m, 1H), 7.26 (s, 1H, Ar-5H), 7.74 (s, 1H, Ar-8H); HR-MS (ESI-positive) : 428.22978 (M+H) (Calculated : 428.22986).

### Example 6. Preparation of Co.6

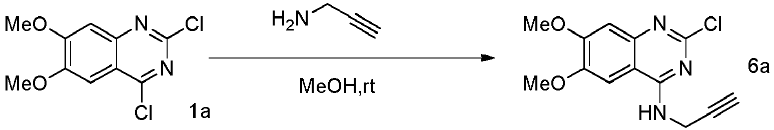

Step 1: Prop-2-yn-1-amine (28 mmol) was added into the solution of compound 1a (20 mmol) in 100 mL methanol, the reaction was carried out at 25 °C for 10 hrs. TLC indicated compound 1a was completely converted, 100 mL of diethyl ether was added, mixed and placed at -20 °C for crystallizing on standing. The resulting white solid was recrystallized with petroleum ether/ethyl acetate to give the final product 6a. Yield: 44%.

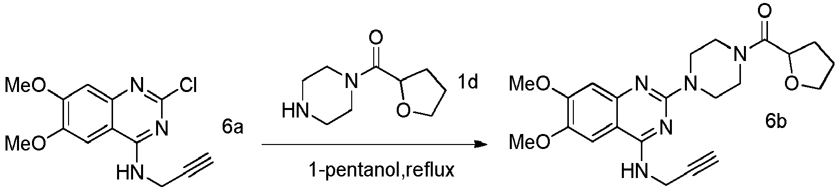

Step 2: Under argon atmosphere, compound 1d (2 mmol) was added to the solution of compound 4a (2 mmol) in 1-pentanol. The reaction mixture was refluxed for 4.5 hrs, placed at 0-5 °C for crystallizing on standing. The resulting white crystals were washed twice with 10mL acetone, and recrystallized with diethyl ether/methanol to give the final product of the compound 6b, Co.6. Yield: 75%.

1H NMR (300 MHz, DMSO-d6): 1.82 (m, 2H, thf-H), 2.03 (m, 2H, thf-H), 3.47-4.07 (m, 6H, thf-H, pip-H), 3.80 (s, 2H), 3.87 (s, 3H, OCH₃), 3.94 (s, 3H, OCH₃), 4.04 (d, 2H), 4.43 (m, 1H, thf-2H), 7.26 (s, 1H, Ar-5H), 7.74 (s, 1H, Ar-8H); HR-MS (ESI-positive) : 426.21413 (M+H) (Calculatd : 426.21408).

### Example 7. Preparation of Co.7

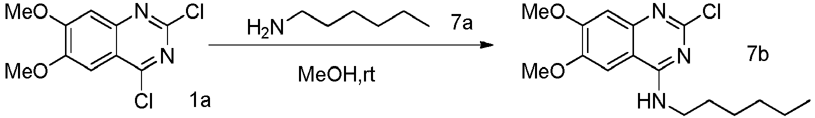

Step 1: Compound 7a (20 mmol) was added into the solution of compound 1a (20 mmol) in 100 mL methanol, the reaction was carried out at 25 °C for 3 hrs. TLC indicated compound 1a was completely converted, 100 mL of diethyl ether was added, mixed and placed at -20 °C for crystallizing on standing. The resulting white solid was recrystallized with petroleum ether/ethyl acetate to give the final product 7b. Yield: 61%.

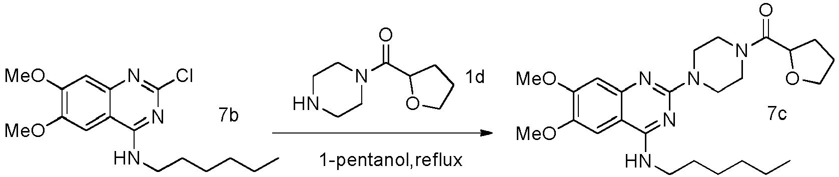

Step 2: Under argon atmosphere, compound 1d (2 mmol) was added to the solution of compound 7b (2 mmol) in 1-pentanol. The reaction mixture was refluxed for 4.5 hrs, placed at 0-5 °C for crystallizing on standing. The resulting white crystals were washed twice with 10mL acetone, and recrystallized with diethyl ether/methanol to give the final product of the compound 7c, Co.7. Yield: 62%.

1H NMR (300 MHz, DMSO-d6): 0.86-1.62 (m, 11H,), 1.84 (m, 2H, thf-H), 2.03 (m, 2H, thf-H), 3.46-4.07 (m, 8H, thf-H, pip-H), 3.86 (s, 3H, OCH₃), 3.83(s, 3H, OCH₃), 4.75 (m, 1H, thf-2H), 7.16 (s, 1H, Ar-5H), 7.72 (s, 1H, Ar-8H); HR-MS (ESI-positive): 472.29238 (M+H) (Calculated : 472.29229).

### Example 8. Preparation of Co.8

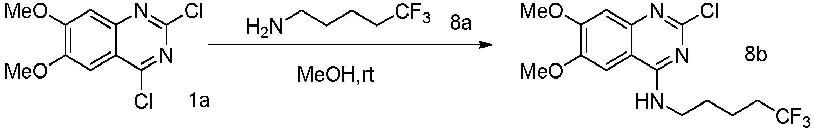

Step 1: Compound 8a (20 mmol) was added into the solution of compound 1a (20 mmol) in 100 mL methanol, the reaction was carried out at 25 °C for 1.5 hrs. TLC indicated compound 1a was completely converted, 100 mL of diethyl ether was added, mixed and placed at -20 °C for crystallizing on standing. The resulting white solid was recrystallized with petroleum ether/ethyl acetate to give the final product 8b. Yield: 71%.

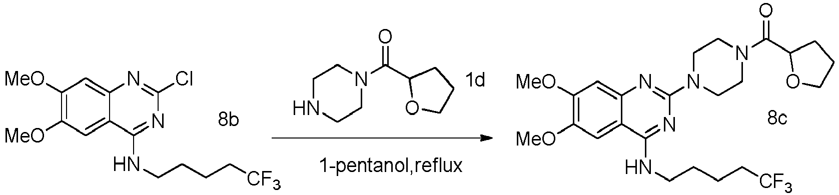

Step 2: Under argon atmosphere, compound 1d (2 mmol) was added to the solution of compound 8b (2 mmol) in 1-pentanol. The reaction mixture was refluxed for 4.5 hrs, placed at 0-5 °C for crystallizing on standing. The resulting white crystals were washed twice with 10mL acetone, and recrystallized with diethyl ether/methanol to give the final product is the compound 8c, Co.8. Yield: 42%.

1H NMR (300 MHz, DMSO-d6): 1.22-1.72 (m, 11H, ) , 1.84(m,2H, thf-H), 2.03 (m, 2H, thf-H), 3.47-4.05 (m, 8H, thf-H, pip-H), 3.82(s, 3H, OCH₃), 3.73 (s, 3H, OCH₃), 4.43 (m, 1H, thf-2H), 7.54 (s, 1H, Ar-5H), 7.86 (s, 1H, Ar-8H); HR-MS (ESI-positive): 512.24846 (M+H) (Calculated: 512.24859).

### Example 9. Preparation of Co.9

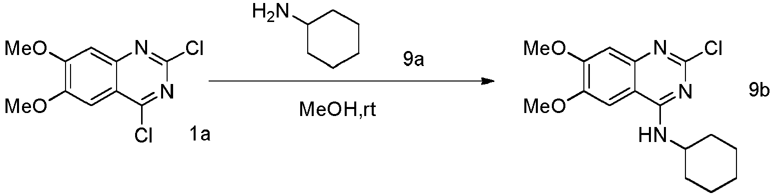

Step 1: Compound 9a (20 mmol) was added into the solution of compound 1a (20 mmol) in 100 mL methanol, the reaction was carried out at 25 °C for 2.5 hrs. TLC indicated compound 1a was completely converted, 100 mL of diethyl ether was added, mixed and placed at -20 °C for crystallizing on standing. The resulting white solid was recrystallized with petroleum ether/ethyl acetate to give the final product 9b. Yield: 21%.

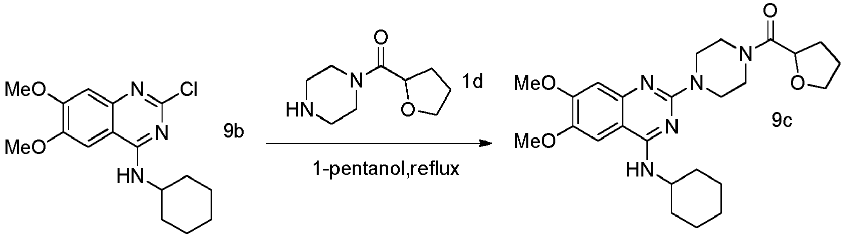

Step 2: Under argon atmosphere, compound 1d (2 mmol) was added to the solution of compound 9b (2 mmol) in 1-pentanol. The reaction mixture was refluxed for 4.5 hrs, placed at 0-5 °C for crystallizing on standing. The resulting white crystals were washed twice with 10mL acetone, and recrystallized with diethyl ether/methanol to give the final product of the compound 9c, Co.9. Yield: 43%.

1H NMR (300 MHz, DMSO-d6): 1.32-1.52 (m, 10H, ), 1.86 (m, 2H, thf-H), 2.03 (m 2H, thf-H), 3.47-4.07 (m, 9H, thf-H, pip-H), 3.82 (s, 3H, OCH₃), 3.79 (s, 3H, OCH₃), 4.46 (m, 1H, thf-2H), 7.33 (s, 1H, Ar-5H), 7.78(s, 1H, Ar-8H); HR-MS (ESI-positive): 470.27673 (M+H) (Calculated : 470.27658).

### Example 10. Preparation of Co.10

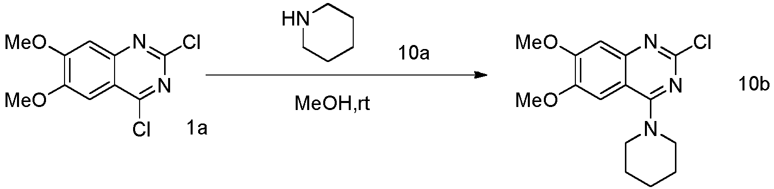

Step 1: Compound 10a (20 mmol) was added into the solution of compound 1a (20 mmol) in 100 mL methanol, the reaction was carried out at 25 °C for 5 hrs. TLC indicated compound 1a was completely converted, 100 mL of diethyl ether was added, mixed and placed at -20 °C for crystallizing on standing. The resulting white solid was recrystallized with petroleum ether/ethyl acetate to give the final product 9b. Yield: 36%.

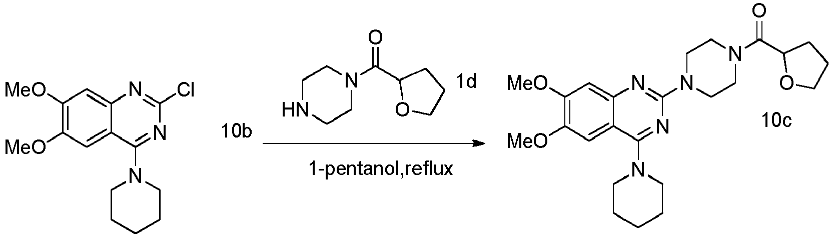

Step 2: Under argon atmosphere, compound 1d (2 mmol) was added to the solution of compound 9b (2 mmol) in 1-pentanol. The reaction mixture was refluxed for 4.5 hrs, placed at 0-5 °C for crystallizing on standing. The resulting white crystals were washed twice with 10mL acetone, and recrystallized with diethyl ether/methanol to give the final product of the compound 10c, Co.10. Yield: 43%.

1H NMR (300 MHz, DMSO-d6): 1.33-1.47 (m, 6H,) , 1.86(m,2H, thf-H), 2.04 (m, 2H, thf-H), 3.57-4.09 (m, 12H, thf-H, pip-H), 3.82 (s, 3H, OCH₃), 3.77 (s, 3H, OCH₃), 4.48 (m, 1H, thf-2H), 7.37 (s, 1H, Ar-5H), 7.79(s, 1H, Ar-8H); HR-MS (ESI-positive): 456.26108 (M+H) (Calculated : 456.26119).

### Example 11. Preparation of Co.11

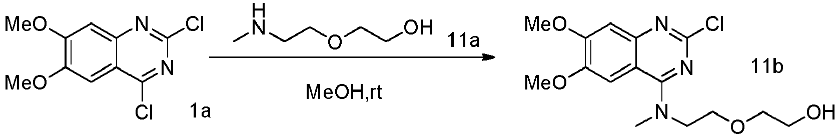

Step 1: Compound 11a (20 mmol) was added into the solution of compound 1a (20 mmol) in 100 mL methanol, the reaction was carried out at 25 °C for 5 hrs. TLC indicated compound 1a was completely converted, 100 mL of diethyl ether was added, mixed and placed at -20 °C for crystallizing on standing. The resulting white solid was recrystallized with petroleum ether/ethyl acetate to give the final product 11c. Yield: 41%.

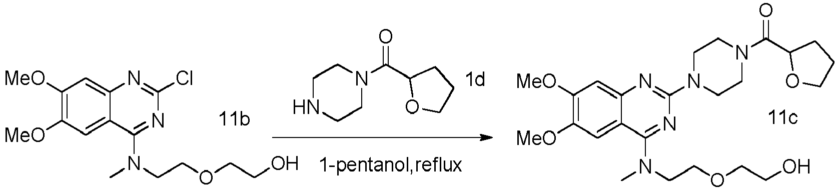

Step 2: Under argon atmosphere, compound 1d (2 mmol) was added to the solution of compound 11b (2 mmol) in 1-pentanol. The reaction mixture was refluxed for 4.5 hrs, placed at 0-5 °C for crystallizing on standing. The resulting white crystals were washed twice with 10mL acetone, and recrystallized with diethyl ether/methanol to give the final product of the compound 11c, Co.11. Yield: 62%.

1H NMR (300 MHz, DMSO-d6): 1.79 (m, 2H, thf-H), 2.06 (m, 2H, thf-H), 3.27-4.08 (m, 17H, thf-H, pip-H 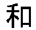 CH₂CH₂O), 3.85 (s, 3H, OCH₃), 3.88 (s, 3H, OCH₃),4.73(m, 1H, thf-2H), 7.26 (s, 1H, Ar-5H), 7.72 (s, 1H, Ar-8H); HR-MS (ESI-positive): 490.26656 (M+H) (Calculated: 490.26658).

### Example 12. Preparation of Co.12

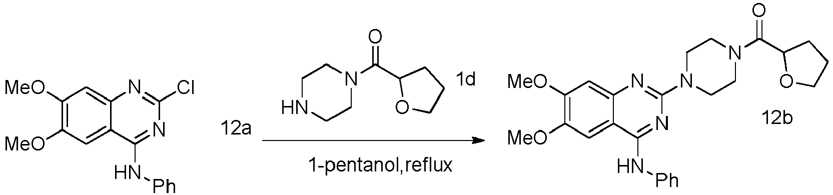

Step 1: Under argon atmosphere, compound 1d (2 mmol) was added to the solution of compound 12a (2 mmol) in 1-pentanol. The reaction mixture was refluxed for 4.5 hrs, placed at 0-5 °C for crystallizing on standing. The resulting white crystals were washed twice with 10mL acetone, and recrystallized with diethyl ether/methanol to give the final product of the compound 12b, Co.12. Yield: 62%.

1H NMR (300 MHz, DMSO-d6): 1.79 (m, 2H, thf-H), 2.06 (m, 2H, thf-H), 3.47-4.08 (m, 11H, thf-H, pip-H), 3.85 (s, 3H, OCH₃), 3.88 (s, 3H, OCH₃), 4.73 (m, 1H, thf-2H), 6.86 -7.62 (m, 7H, Ar-H); HR-MS (ESI-positive): 464.22978 (M+H) (Calculated: 464.22996).

### Example 13. Preparation of Co.13

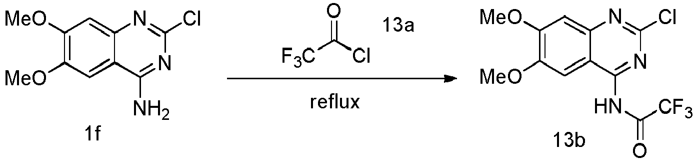

Step 1: Compound 1f (10mmol) was dissolved in 20mL compound 13a, the reaction mixture was refluxed for 2 hrs, cooled to room temperature, a lot of white solid precipitated and was filtered, and the resulting white solid was washed with tetrahydrofuran to give the final product 13b. Yield: 82%.

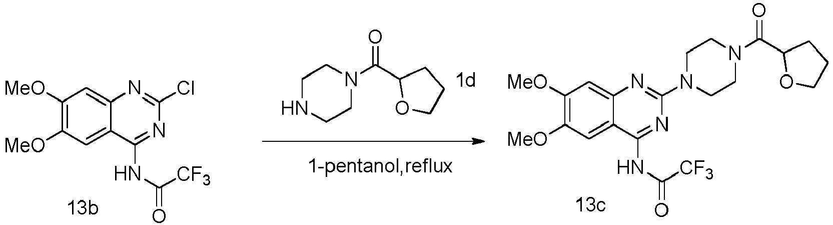

Step 2: Under argon atmosphere, compound 1d (2 mmol) was added to the solution of compound 13b (2 mmol) in 1-pentanol. The reaction mixture was refluxed for 5.5 hrs, placed at 0-5 °C for crystallizing on standing. The resulting white crystals were washed twice with 10mL acetone, and recrystallized with diethyl ether/methanol to give the final product of the compound 13c, Co.13. Yield: 62%.

1H NMR (300 MHz, CDCl₃): 1.80 (m, 2H, thf-H), 2.06 (m, 2H, thf-H), 3.47-4.07 (m, 13H, thf-H , pip-H), 3.93 (s, 3H, OCH₃), 3.97 (s, 3H, OCH₃), 4.82 (m, 1H, thf-2H), 7.16 (s, 1H, Ar-5H), 7.72 (s, IH, Ar-8H); HR-MS (ESI-positive): 484.18078 (M+H) (Calculated: 484.18084).

### Example 14. Preparation of Co.14

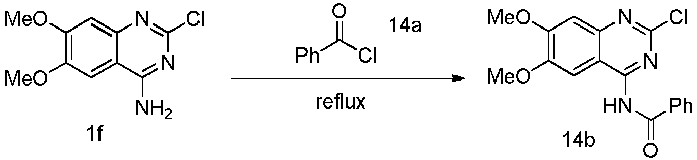

Step 1: Compound 1f (10mmol) was dissolved in 22mL compound 14a, the reaction mixture was refluxed for 2 hrs, cooled to room temperature, a lot of white solid precipitated and was filtered, and the resulting white solid was washed with tetrahydrofuran to give the final product 14b. Yield: 55%.

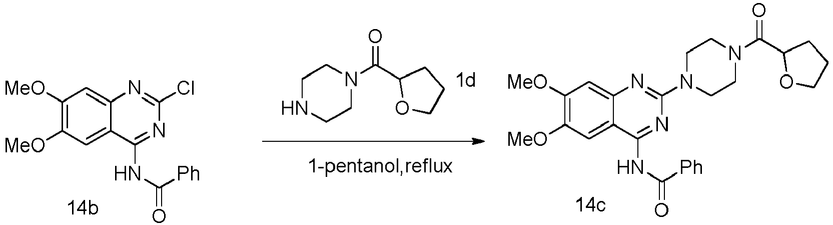

Step 2: Under argon atmosphere, compound 1d (2 mmol) was added to the solution of compound 14b (2 mmol) in 1-pentanol. The reaction mixture was refluxed for 4.5 hrs, placed at 0-5 °C for crystallizing on standing. The resulting white crystals were washed twice with 10mL acetone, and recrystallized with diethyl ether/methanol to give the final product of the compound 14c, Co.14. Yield: 48%.

1H NMR (300 MHz, CDCl₃): d 1.84 (m, 2H, thf-H), 2.03 (m, 2H, thf-H), 3.45-4.47 (m, 13H, thf-H, pip-H), 3.93 (s, 3H, OCH₃), 3.97 (s, 3H, OCH₃), 4.82 (m, 1H, thf-2H), 6.89 -7.72 (m, 7H, Ar-H); HR-MS (ESI-positive) : 492.22469 (M+H) (Calculated: 492.22478).

### Example 15. Preparation of Co.15

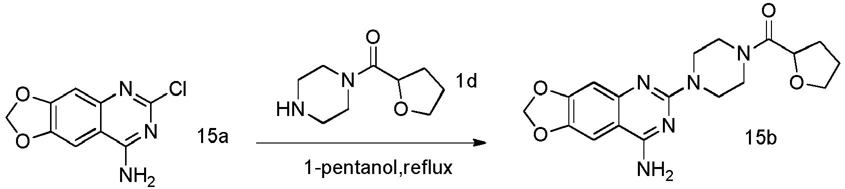

Step 1: Under argon atmosphere, compound 1d (2 mmol) was added to the solution of compound 15b (2 mmol) in 1-pentanol. The reaction mixture was refluxed for 4.5 hrs, placed at 0-5 °C for crystallizing on standing. The resulting white crystals were washed twice with 10mL acetone, and recrystallized with diethyl ether/methanol to give the final product of the compound 15c, Co.15. Yield: 55%.

1H NMR (300 MHz, CDCl₃): 1.84 (m, 2H, thf-H), 2.03 (m, 2H, thf-H), 3.49-4.27 (m, 13H, thf-H, pip-H), 4.82 (m, 1H, thf-2H), 6.07(s, 2H, CH₂OCH₂)7.27(s, 1H , Ar-5H), 7.68 (s, 1H, Ar-8H); HR-MS (ESI-positive) : 372.16718 (M+H) (Calculated: 372.16726).

### Example 16. Preparation of Co.16

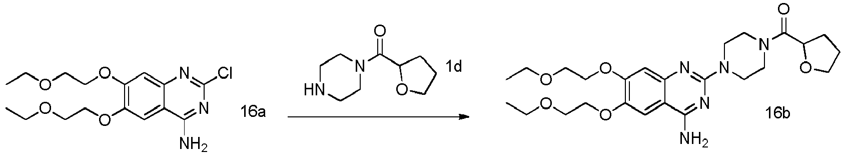

Step 1: Under argon atmosphere, compound 1d (2 mmol) was added to the solution of compound 16a (2 mmol) in 1-pentanol. The reaction mixture was refluxed for 4.5 hrs, placed at 0-5 °C for crystallizing on standing. The resulting white crystals were washed twice with 10mL acetone, and recrystallized with diethyl ether/methanol to give the final product of the compound 16a, Co.16. Yield: 45%.

1H NMR (300 MHz, CDCl₃): 1.15-1.26 (t, 6H, CH₃-H), 1.87(m, 2H, thf-H), 2.03 (m, 2H, thf-H), 3.45-4.17 (m, 25H, thf-H, pip-H), 4.79 (m, 1H, thf-2H), 7.27(s, 1H, Ar-5H), 7.68 (s, 1H, Ar-8H); HR-MS (ESI-positive) : 504.28215 (M+H) (Calculated: 504.28221).

### Example 17. Preparation of Co.17

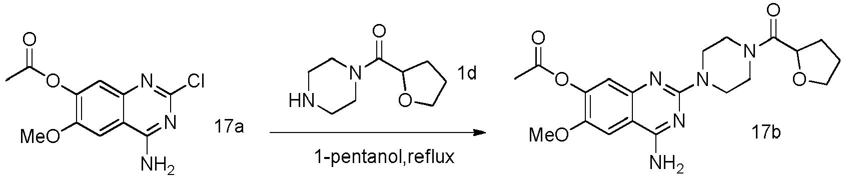

Step 1: Under argon atmosphere, compound 1d (2 mmol) was added to the solution of compound 17a (2 mmol) in 1-pentanol. The reaction mixture was refluxed for 4 hrs, placed at 0-5 °C for crystallizing on standing. The resulting white crystals were washed twice with 10mL acetone, and recrystallized with diethyl ether/methanol to give the final product is the compound 17a, Co.17. Yield: 55%.

1H NMR (300 MHz, CDCl₃): 1.83 (m, 2H, thf-H), 2.03 (m, 2H, thf-H), d2.61 (s,3H , CH₃), 3.47-4.07 (m, 11H, thf-H, pip-H 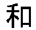 CH₂CH₂O), 3.94 (s, 3H, OCH₃), 4.82 (m 1H , thf-2H), 7.18 (s, 1H, Ar-5H), 7.72 (s, 1H, Ar-8H); HR-MS (ESI-positive) : 416.19344 (M+H) (Calculated: 416.19339).

### Example 18 preparation of Co.18

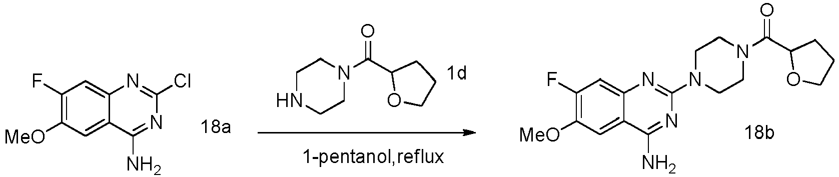

Step 1: Under argon atmosphere, compound 1d (2 mmol) was added to the solution of compound 18a (2 mmol) in 1-pentanol. The reaction mixture was refluxed for 3 hrs, placed at 0-5 °C for crystallizing on standing. The resulting white crystals were washed twice with 10mL acetone, and recrystallized with diethyl ether/methanol to give the final product of the compound 18b, Co.18. Yield: 75%.

1H NMR (300 MHz, CDCl₃): 1.80 (m, 2H, thf-H), 2.03 (m, 2H, thf-H), 3.57-4.07 (m, 11H, thf-H, pip-H), 3.94 (s, 3H, OCH₃), 4.85 (m, 1H, thf-2H), 7.23 (s, 1H, Ar-5H), 7.80 (s, 1H, Ar-8H); HR-MS (ESI-positive) : 376.17853 (M+H) (Calculated: 376.17849).

### Example 19. Preparation of Co.19

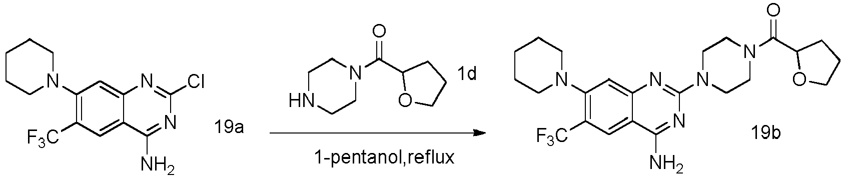

Step 1: Under argon atmosphere, compound 1d (2 mmol) was added to the solution of compound 19a (2 mmol) in 1-pentanol. The reaction mixture was refluxed for 3 hrs, placed at 0-5 °C for crystallizing on standing. The resulting white crystals were washed twice with 10mL acetone, and recrystallized with diethyl ether/methanol to give the final product of the compound 19b, Co.19. Yield: 54%.

1H NMR (300 MHz, CDCl₃): 1.15-1.26 (m, 6H), 1.89 (m, 2H, thf-H), 2.03(m, 2H, thf-H), 3.07-4.07 (m, 15H, thf-H, pip-H), 4.85 (m, 1H, thf-2H), 7.63 (s, 1H, Ar-5H), 7.89 (s, 1H, Ar-8H); HR-MS (ESI-positive) : 479.23836 (M+H) (Calculated: 479.23823).

### Example 20. Preparation of Co.20

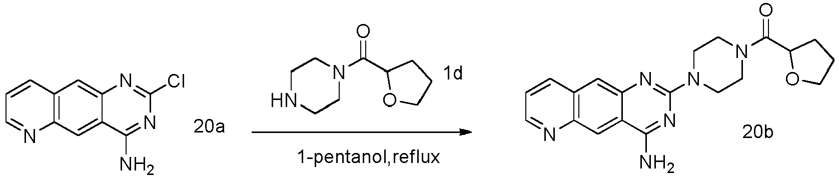

Step 1: Under argon atmosphere, compound 1d (2 mmol) was added to the solution of compound 20a (2 mmol) in 1-pentanol. The reaction mixture was refluxed for 3 hrs, placed at 0-5 °C for crystallizing on standing. The resulting white crystals were washed twice with 10mL acetone, and recrystallized with diethyl ether/methanol to give the final product of the compound 20b, Co.20. Yield: 54%.

1H NMR (300 MHz, CDCl₃): 1.83 (m, 2H, thf-H), 2.03 (m, 2H, thf-H), 3.07-4.07 (m, HH, thf-H, pip-H), 4.88 (m, 1H, thf-2H), 7.58 (m, IH, Ar-H), 7.63 (s, IH, Ar-H), 7.89 (s, 1H, Ar-H), 8.38(d, 1H, Ar-H) 8.83(d, 1H, Ar-H); HR-MS (ESI-positive) : 379.18833 (M+H) (Calculated: 379.18825).

### Example 21. Preparation of Co.21

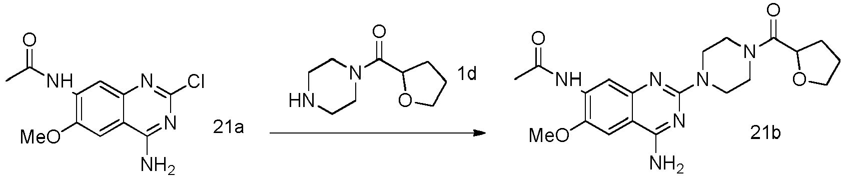

Step 1: Under argon atmosphere, compound 1d (2 mmol) was added to the solution of compound 21 a (2 mmol) in 1-pentanol. The reaction mixture was refluxed for 4 hrs, placed at 0-5 °C for crystallizing on standing. The resulting white crystals were washed twice with 10mL acetone, and recrystallized with diethyl ether/methanol to give the final product of the compound 21b, Co.21. Yield: 54%.

1H NMR (300 MHz, CDCl₃): 1.86 (m, 2H, thf-H), 2.01 (m, 2H, thf-H), 2.62 (s, 3H, CH₃), 3.17 - 4.07 (m, 11H, thf-H, pip-H), 3.97 (s, 3H, OCH₃), 4.85 (m, 1H, thf-2H), 7.89 (s, 1H, Ar-H), 8.83(d, 1H, Ar-H); HR-MS (ESI-positive) : 379.18833 (M+H) (Calculated: 379.18825).

### Example 22. Preparation of Co.22

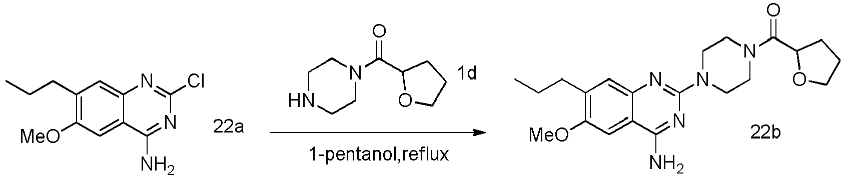

Step 1: Under argon atmosphere, compound 1d (2 mmol) was added to the solution of compound 22a (2 mmol) in 1-pentanol. The reaction mixture was refluxed for 5 hrs, placed at 0-5 °C for crystallizing on standing. The resulting white crystals were washed twice with 10mL acetone, and recrystallized with diethyl ether/methanol to give the final product of the compound 22b, Co.22. Yield: 61%.

1H NMR (300 MHz, CDCl₃): 0.88-0.93(t, 3H), 1.62(m, 2H), 1.88(m, 2H, thf-H), 2.03 (m, 2H, thf-H), 2.64(t, 2H), 3.57-4.07 (m, 11H, thf-H, pip-H), 3.94 (s, 3H, OCH₃), 4.85 (m, 1H, thf-2H), 7.23 (s, 1H, Ar-5H), 7.82 (s, 1H, Ar-8H); HR-MS (ESI-positive): 400.23464 (M+H) (Calculated: 400.23486).

### Example 23. Preparation of Co.23

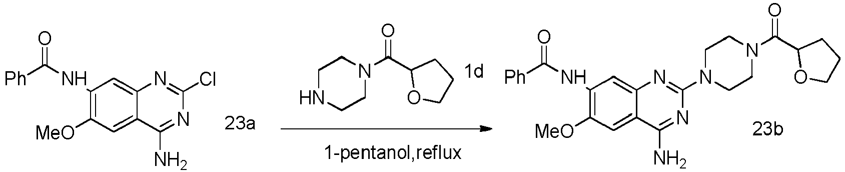

Step 1: Under argon atmosphere, compound 1d (2 mmol) was added to the solution of compound 23a (2 mmol) in 1-pentanol. The reaction mixture was refluxed for 5 hrs, placed at 0-5 °C for crystallizing on standing. The resulting white crystals were washed twice with 10mL acetone, and recrystallized with diethyl ether/methanol to give the final product of the compound 23b, Co.23. Yield: 48%.

1H NMR (300 MHz, CDCl₃): 1.87 (m, 2H, thf-H), 2.01 (m, 2H, thf-H), 3.17-4.07 (m, 11H, thf-H, pip-H), 3.97 (s, 3H, OCH₃), 4.87 (m, 1H, thf-2H), 6.86 -7.62 (m, 7H, Ar-H); HR-MS (ESI-positive) : 477.22497 (M+H) (Calculated: 477.22503).

### Example 24 preparation of Co.24

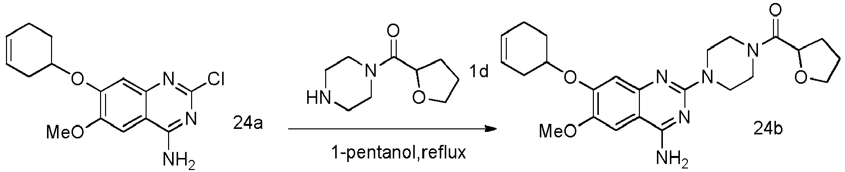

Step 1: Under argon atmosphere, compound 1d (2 mmol) was added to the solution of compound 25a (2 mmol) in 1-pentanol. The reaction mixture was refluxed for 4 hrs, placed at 0-5 °C for crystallizing on standing. The resulting white crystals were washed twice with 10mL acetone, and recrystallized with diethyl ether/methanol to give the final product is the compound 25b, Co.25. Yield: 24%.

1H NMR (300 MHz, CDCI₃): 1.86 (m, 2H, thf-H), 1.94-2.58 (m, 8H), 3.17-4.07 (m, 12H, thf-H, pip-H), 3.97 (s, 3H, OCH3), 4.85 (m, 1H, thf-2H), 5.56 (m, 2H), 7.15 (s, 1H, Ar-5H), 7.71 (s, 1H, Ar-8H); HR-MS (ESI-positive) : 454.24499 (M+H) (Calculated: 454.24543).

### Example 25. Preparation of Co.25

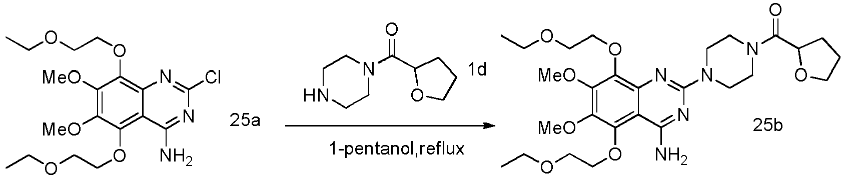

Step 1: Under argon atmosphere, compound 1d (2 mmol) was added to the solution of compound 25a (2 mmol) in 1-pentanol. The reaction mixture was refluxed for 4.5 hrs, placed at 0-5 °C for crystallizing on standing. The resulting white crystals were washed twice with 10mL acetone, and recrystallized with diethyl ether/methanol to give the final product is the compound 25b, Co.25. Yield: 45%.

1H NMR (300 MHz, CDCl₃): 1.15-1.26 (t, 6H, CH₃-H) 1.88 (m, 2H, thf-H), 2.03 (m, 2H, thf-H), 3.44-4.17 (m, 25H, thf-H, pip-H), 3.85 (s, 3H, OCH₃), 3.87 (s, 3H, OCH₃),4.87 (m, 1H, thf-2H), 7.27 (s, 1H, Ar-5H), 7.68 (s, 1H, Ar-8H); HR-MS (ESI-positive) : 564.30330 (M+H) (Calculated: 564.30334).

### Example 26. Preparation of Co.26

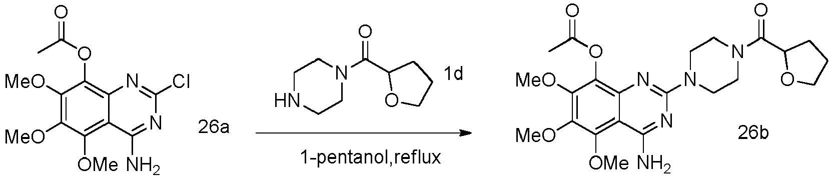

Step 1: Under argon atmosphere, compound 1d (2 mmol) was added to the solution of compound 26a (2 mmol) in 1-pentanol. The reaction mixture was refluxed for 4 hrs, placed at 0-5 °C for crystallizing on standing. The resulting white crystals were washed twice with 10mL acetone, and recrystallized with diethyl ether/methanol to give the final product is the compound 26a, Co.17. Yield: 55%.

1H NMR (300 MHz, CDCl₃): 1.86 (m, 2H, thf-H), 2.03 (m, 2H, thf-H), d 2.61 (s, 3H, CH₃), 3.47-4.07 (m, HH, thf-H, pip-H 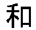 CH₂CH₂O), 3.91 (s, 3H, OCH₃), 3.95 (s, 3H, OCH₃), 3.99(s, 3H, OCH₃), 4.82(m, 1H, thf-2H), 7.16(s, 1H, Ar-5H), 7.72 (s, 1H, Ar-8H); HR-MS (ESI-positive) : 476.21445 (M+H) (Calculated: 476.21452).

### Example 27. Preparation of Co.27

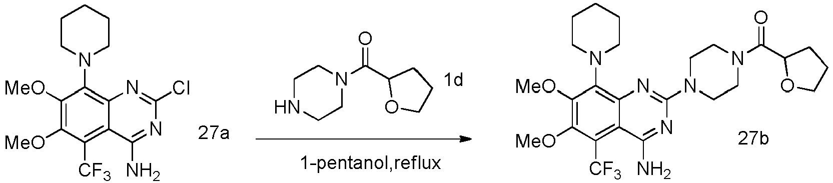

Step 1: Under argon atmosphere, compound 1d (2 mmol) was added to the solution of compound 27a (2 mmol) in 1-pentanol. The reaction mixture was refluxed for 3 hrs, placed at 0-5 °C for crystallizing on standing. The resulting white crystals were washed twice with 10mL acetone, and recrystallized with diethyl ether/methanol to give the final product is the compound 27b, Co.27. Yield: 53%.

1H NMR (300 MHz, CDCl₃): 1.15-1.26(m, 6H), 1.88(m, 2H, thf-H), 2.03 (m, 2H, thf-H), 3.07-4.07 (m, 15H, thf-H, pip-H), 3.86 (s, 3H, OCH₃), 3.88 (s, 3H, OCH₃), 4.85 (m, 1H, thf-2H), 7.63 (s, 1H, Ar-5H), 7.89 (s, 1H, Ar-8H); HR-MS (ESI-positive) : 539.25942 (M+H) (Calculated: 539.25936).

### Example 28. Preparation of Co.28

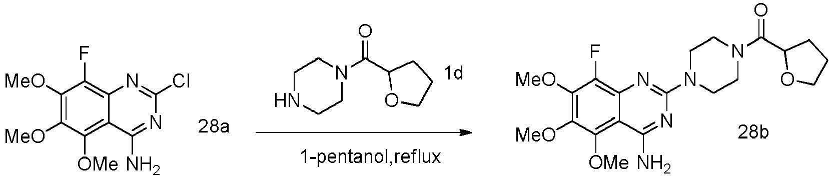

Step 1: Under argon atmosphere, compound 1d (2 mmol) was added to the solution of compound 28a (2 mmol) in 1-pentanol. The reaction mixture was refluxed for 3 hrs, placed at 0-5 °C for crystallizing on standing. The resulting white crystals were washed twice with 10mL acetone, and recrystallized with diethyl ether/methanol to give the final product is the compound 28b, Co.28. Yield: 75%.

1H NMR (300 MHz, CDCl₃): 1.78 (m, 2H, thf-H), 2.03 (m, 2H, thf-H), 3.57-4.07 (m, 11H, thf-H, pip-H), 3.86 (s, 3H, OCH₃), 3.88 (s, 3H, OCH₃), 3.94 (s, 3H, OCH₃), 4.85 (m, 1H, thf-2H), 7.23 (s, 1H, Ar-5H), 7.82 (s, 1H, Ar-8H); HR-MS (ESI-positive): 436.19955 (M+H) (Calculated: 436.19962).

### Example 29. Preparation of Co.29

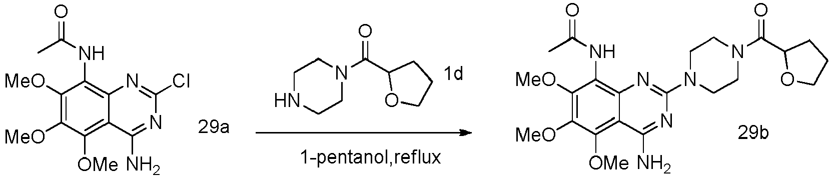

Step 1: Under argon atmosphere, compound 1d (2 mmol) was added to the solution of compound 29a (2 mmol) in 1-pentanol. The reaction mixture was refluxed for 3 hrs, placed at 0-5 °C for crystallizing on standing. The resulting white crystals were washed twice with 10mL acetone, and recrystallized with diethyl ether/methanol to give the final product is the compound 29b, Co.29. Yield: 44%.

1H NMR (300 MHz, CDCl₃): 1.84 (m, 2H, thf-H), 2.01 (m, 2H, thf-H), 2.62 (s, 3H, CH₃), 3.17-4.07 (m, 11H, thf-H, pip-H), 3.82 (s, 3H, OCH₃), 3.85 (s, 3H, OCH₃), 3.97 (s, 3H, OCH₃), 4.85 (m, 1H, thf-2H), 7.89 (s, 1H, Ar-H), 8.83(d, 1H, Ar-H); HR-MS (ESI-positive) : 475.23050 (M+H) (Calculated: 475.23051).

### Example 30. Preparation of Co.30

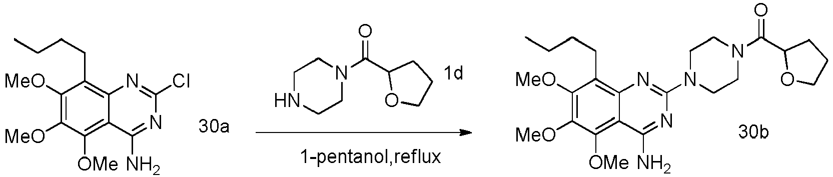

Step 1: Under argon atmosphere, compound 1d (2 mmol) was added to the solution of compound 30a (2 mmol) in 1-pentanol. The reaction mixture was refluxed for 5 hrs, placed at 0-5 °C for crystallizing on standing. The resulting white crystals were washed twice with 10mL acetone, and recrystallized with diethyl ether/methanol to give the final product is the compound 30b, Co.30. Yield: 65%.

1H NMR (300 MHz, CDCl₃): 0.88-0.93(t, 3H), 1.62(m, 2H), 1.84 (m, 2H, thf-H), 2.03 (m, 2H, thf-H), 2.64(t, 2H), 3.57-4.07 (m, 11H, thf-H, pip-H), 3.71 (s, 3H, OCH₃), 3.76 (s, 3H, OCH₃), 3.91 (s, 3H, OCH₃), 4.85 (m, 1H, thf-2H), 7.23 (s, 1H, Ar-5H), 7.82 (s, 1H, Ar-8H); HR-MS (ESI-positive) : 400.23464 (M+H) (Calculated: 474.27164).

### Example 31 preparation of Co.31

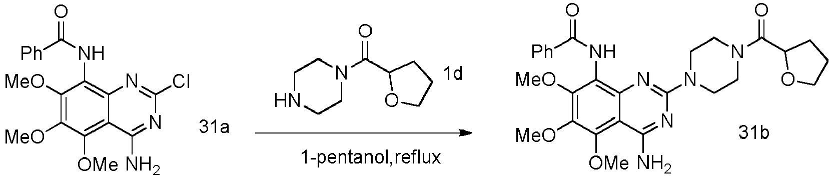

Step 1: Under argon atmosphere, compound 1d (2 mmol) was added to the solution of compound 31 a (2 mmol) in 1-pentanol. The reaction mixture was refluxed for 4 hrs, placed at 0-5 °C for crystallizing on standing. The resulting white crystals were washed twice with 10mL acetone, and recrystallized with diethyl ether/methanol to give the final product is the compound 31b, Co.31. Yield: 24%.

1H NMR (300 MHz, CDCl₃): 1.864 (m, 2H, thf-H), 1.93-2.58 (m, 8H), 3.17-4.07 (m, 12H, thf-H, pip-H), 3.80 (s, 3H, OCH₃), 3.83 (s, 3H, OCH₃), 3.94 (s, 3H, OCH₃), 4.85 (m, 1H, thf-2H), 5.56 (m, 2H), 7.15 (s, 1H, Ar-5H), 7.71 (s, 1H, Ar-8H); HR-MS (ESI-positive) : 536.25085 (M+H) (Calculated: 536.25091).

### Example 32. Preparation of Co.32

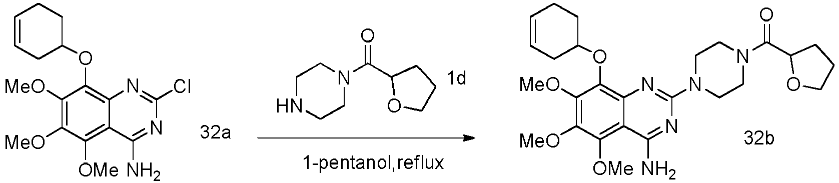

Step 1: Under argon atmosphere, compound 1d (2 mmol) was added to the solution of compound 32a (2 mmol) in 1-pentanol. The reaction mixture was refluxed for 4 hrs, placed at 0-5 °C for crystallizing on standing. The resulting white crystals were washed twice with 10mL acetone, and recrystallized with diethyl ether/methanol to give the final product is the compound 32b, Co.32. Yield: 45%.

1H NMR (300 MHz, CDCl₃): 1.860 (m, 2H, thf-H), 1.95-2.58 (m, 8H), 3.17-4.07 (m, 12H, thf-H , pip-H), 3.80 (s, 3H, OCH₃), 3.86 (s, 3H, OCH₃), 3.93 (s, 3H, OCH₃), 4.85 (m, 1H, thf-2H), 5.56 (m, 2H), 7.15 (s, 1H, Ar-5H), 7.71 (s, 1H, Ar-8H); HR-MS (ESI-positive) : 514.26655 (M+H) (Calculated: 514.26656).

### B. Biological experiments

### 1. Fly stocks and compound screen

*UAS-rpr* and *HS-Gal4* flies were obtained from Bloomington Stock Center in the US. The F1 progenies (*HS>rpr*) from the cross of these two lines were raised at 18 °C for the compound screen. Compounds were dissolved in 5% sucrose at the concentrations used in the culture media for Cmap. The compound solution (150 µL) was added to a vial containing three layers of filter papers. Twenty adult *HS>rpr* flies aged 1-3 days were then placed into each vial for 24 hours. The flies were heat shocked at 37 °C for 2 hrs. The survival rate was calculated 12 hrs after the heat shock.

### 2. Microarray processing and data analysis

Ten vials of *HS>rpr* flies and progenies of *HS-Gal4* and *yw67c23* (a genetic-background-matched control line) aged 1-3 days were collected. Each vial contained 35 female flies. The flies were frozen in liquid nitrogen before heat shock (as time 0), and after heat shock at 1, 2, 3, 4, 5, 6, 7, 8 and 12 hours. Total RNA was prepared by RNeasy^{®} mini kit (Qiagen) and further processed by TURBO™ DNase kit (Ambion, Inc) to remove chromosomal DNA from the samples. Quality control of the RNA samples and microarray processing procedures were performed according to the standard protocols for Affymetrix DNA microarrays (Affymetrix, Inc). Drosophila 2.0 DNA microarrays from Affymetrix were used (Affymetrix, Inc). Data analysis was performed using the Arrayassist 5.0 (Affymetrix, Inc). The algorithm of "RMA" was used to analyze the data. Because only one experimental sample was collected for each time point, time points from three sequential samples were grouped for analysis, the mean value of these time points was determined to be the data point for each group. For example, the time points 0, 1 and 2 were grouped as "time point 1." In the present invention, the experimental design aimed to increase the temporal resolution of gene expression changes during apoptosis. It was hypothesized that transcriptional changes should be continuous and reflected between adjacent time points. For gene ontology enrichment, an online tool (http://david.abcc.ncifcrf.gov/) was used.

### 3. Exploration of candidate compounds by Cmap

The up-regulated and down-regulated genes from the Drosophila microarray were converted to mammalian homologous genes based on an online database (www.affymetrix.com). The signature files were then generated based on the protocol provided by Cmap (Lamb et al., 2006), and the list of predicted compounds with the best positive or negative correlations was generated. The compounds were purchased from Sigma-Aldrich.

### 4. Cell culture and apoptosis induction

The RAW 264.7 cells were cultured in the DMEM medium (Gibco) supplemented with 10% FBS (Gibco), streptomycin (0.1 g/L) and penicillin (0.06 g/L) (Amresco). To induce apoptosis, RAW 264.7 cells at 90% confluence were incubated in DMEM (10% FBS) containing 2 µg/mL lipopolysaccharide (LPS; *Escherichia coli* O111:B4, Sigma) for 24 hours.

### 5. Hoechst staining

Cells were fixed in Carnoy's solution (75% EtOH, 25% EtAc) and then stained with Hoechst 33342 (10 µg/mL, Sigma) for 10 min. The cells were washed with ice cold PBS for three times and imaged under a confocal microscope (TCS SP5, Leica).

### 6. Mouse models of sepsis

Male BALB/C mice (20±3g) from Vital River (Beijing, China) were housed in the Animal Center of Peking University. The animal studies were approved by the Institutional Animal Care and Use Committee (IACUC) of Peking University. For the LPS model of sepsis, each mouse was injected with LPS (13.5 mg/kg, i.p.) once. For *E. coli* induced sepsis model, mice were administrated 1 x 10⁸ CFU E. coli by i.p. injection. Terazosin (0.4 mg/kg or 0.04 mg/kg) or saline was then injected (i.p.) 1.5 hours after LPS infusion. Lethality was recorded every 12 hours for 7 days.

For the CLP model of sepsis, mice were anesthetized with chloral hydrate (300 mg/kg, i.p.). Then, a 1.5-2.0 cm long abdominal midline incision was made and the cecum was exposed. After ligation at 5 mm from the cecal tip, the cecum was punctured once with a 22-gauge needle. The abdomen was closed by continuous suture, and the animal recovered with injection of pre-warmed saline (37 °C; 5 ml/100g, s.c.). Co-Am (Lunan Pharmaceutical, Shandong, China) (30 mg/kg) was intragastrically administered for antibiotic therapy. Lethality was recorded every 12 hours for 7 days.

### 7. Western blot

For Western blot, an anti-active Caspase 3 antibody (#9664, Cell Signaling) was used. The anti-actin antibody was purchased from Wuhan Boster company, China.

### 8. DNA fragmentation analysis

24 hrs after LPS injection, one thymus from each mouse was collected. Then, the genomic DNA of thymus was extracted with a genomic DNA purification kit (Biofuture, Beijing, China). DNA fragmentation was assessed on a DNA agarose gel.

### 9. Analysis of inhibitory activity of bacteria

The antibiotic activity of terazosin was measured by Oxford Cup test. Oxford Cups containing different concentrations of terazosin were placed in the petri dish wherein *E. Coli* O111:B4 was cultured on LB medium. The effects of terazosin and ampicillin on the growth of *E. Coli* were examined after 24 hrs.

### 10. Exploration of terazosin target

Terazosin was immobilized on Affi-Gel beads and mixed with cell lysate of RAW 264.7 cells. Protein impurities bound to the Affi-Gel were removed by washing. The binding proteins were eluted with the sample buffer and analyzed by electrophoresis (15% protein gel) after heated at 96 °C. The specific protein band was cut and identified by mass spectrometry.

### 11. Effect of terazosin on the enzymatic activity of pgk1

The recombinant mouse pgk1 protein (His-pgk1) was expressed in the E. Coli. The protein was purified by a Nickel column. The proteins were diluted to 0.15 unit /mL. Then, different concentrations of terazosin were added to determine the pgk1 activity.

### 12. Affinity assay of terazosin and pgk1

His-pgk1 was incubated with differently processed Affi-Gel, and nonspecifically bound proteins were removed after 4 hrs. The binding proteins were eluted with the sample buffer and analyzed by electrophoresis (15% protein gel) after heated at 96 °C.

### 13. Statistical analyses

The Kaplan-Meier test was used under the Log Rank algorithm for survival analysis. One-way ANOVA test was used for group comparison, and Student-*t* test was used to compare two data sets. Based on the present invention, terazosin can be used as an apoptosis inhibitor. This novel function may be applicable to treat and/or prevent sepsis and its complications thereof.

### 14. Activity assay of pgk1

According to the components of Colorimetric GAPDH Assay Kit (ScienCells), the reaction solution was prepared with solutions of GAPDH, HEPES, and MgSO₄. A predetermined concentration of the compound of Formula I was added to the reaction solution (30 mM Hepes, 3 mM ATP, 0.22 mM NADH, 10 mM MgSO₄, 10 mM 3-PGA, 3-4 U/ml GAPDH, pH 7.5). Absorbance was measured at 25 °C with a spectroscope (340 nm). Relative activity of pgk1 was calculated based on the changes of absorbance value within 1-10 minutes. The activity has a positive correlation with the changes of absorbance value. The absorbance of the same compound at 340 nm was subtracted in the test group.

The activation of pgk1 by the compound of Formula I was represented by fold changes, including terazosin (0.02 µg/ml) to enhance the Pkg1 activity by 2.9 fold; compounds Co.1 to Co.32 (0.02 µg/ml) to activate pgk1 to the levels reported above. For instance, Co. 1 and Co.2 activated pgk1 by 2.9 and 2.3 fold, respectively.

### 15. Induction of apoptosis and measurement

RAW 264.7 cells were cultured in the DMEM medium (Gibco) supplemented with 5% FBS, streptomycin (0.1 mg/mL) and penicillin (0.06 mg/mL). To induce apoptosis, 2 µg/mL lipopolysaccharide (LPS; *Escherichia coli* O111:B4, Sigma-Aldrich) and IFN-γ (50 U/mL, Peprotech) were added, and a predetermined concentration of the testing compound was added at the same time. Cell death was assessed by Western blot with an anti-active Caspase 3 antibody (#9661, Cell Signaling). β-Actin (Boster) was used as internal control for protein loading. The ratio of Caspase 3/β-Actinwas was used to assess apoptosis.

### 16. Terazosin effect on serum glucose

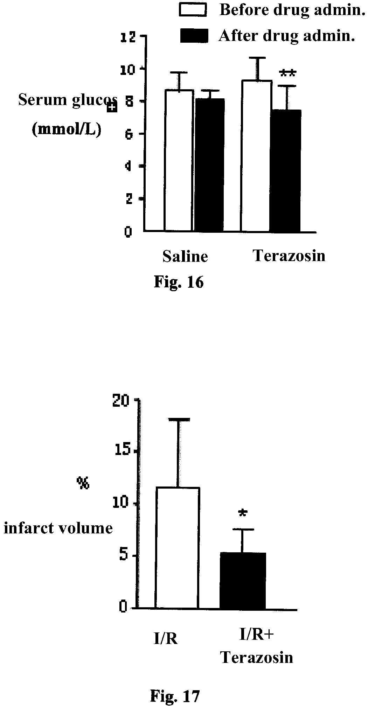

Mice were injected terazosin (0.4 mg/kg i.p.) once. The serum glucose level of the mice was measured 18 hours later. It was found that the serum glucose level was reduced ∼30% (*t*-test, P<0.01); as a control, injection of saline showed no difference. The result is shown in Fig. 16. By the same method, exemplary compounds Co.1, Co.3, Co.7, Co.9, Co.13, Co.16, Co.19, Co.24, Co.27 and Co.32 as described in the present invention were all shown to be effective to reduce the serum glucose level by 20% to 50%.

### 17. Terazosin against cerebral thrombosis

In a mouse model of cerebral thrombosis, the mouse brains were stained after death from thrombosis. White regions representing cell death appeared in the brain issue. After administration of terazosin (0.4 mg/kg i.p.), the area of cell death was significantly reduced. The statistical analysis was performed based on % infarct volume = white area/total area, 6 mice were tested for each condition. t-test, *P*<0.05. As shown in Fig. 17, terazosin could significantly reduce the infarct volume. By the same method, it was found that other exemplary compounds in the present invention, including Co.1, Co.4, Co.7, Co.8, Co.13, Co14, Co.16, Co.19, Co.24-27, Co.29, Co.31, and Co.32, were all shown to be effective to reduce the infarct volume, P< 0.05.

### 18. Effects against sepsis

BALB/C mice (around 20g) was intraperitoneally injected (i.p.) with LPS (13.5 mg/kg). After 1.5 hours, compounds were injected (i.p.) at a predetermined concentration. Mouse survival was examined every 12 hours for 7 days. Statistical significance of the difference in the survival rates was analyzed.

Another model is Cecal Ligation and Puncture (CLP) model of sepsis. Mice were anesthetized. A 1.5-2.0 cm long abdominal midline incision was made and the cecum was exposed. After ligation 5 mm from the cecal tip, the cecum was punctured once with a 22-gauge needle, and some stool was squeezed out. Then the cecum was put back into the abdomen and the abdomen was closed by running suture. After 1.5 hours, compounds were injected (i.p.) at a predetermined concentration. Mouse survival was examined every 12 hours for 7 days. Statistical significance of the difference in the survival rates was analyzed.

The results from the above sepsis models were used to test compounds Co.1, Co.4, Co.9, Co.13, Co.18, Co.21, Co.28, Co.29 and Co.33. These compounds were individually injected (i.p.) once a day for 3 days at 0.4 mg/kg. In the 7-day experimental period, these compounds all significantly increased survival against sepsis (*P* < 0.05, compared to negative controls).

### References:

1. Bone, R.C. Sir Isaac Newton, sepsis, SIRS , and CARS , Crit Care Med. 24, 1125-1128 (1996).
2. Braun, J. S. et al. Neuroprotection by a caspase inhibitor in acute bacterial meningitis. Nat. Med. 5, 298-302 (1999).
3. Hotchkiss, R. S. et at Caspase inhibitors improve survival in sepsis: a critical role of the lymphocyte. Nat. Immunol .1, 496-501 (2000).
4. Chung, C. S. et al. Inhibition of Fas signaling prevents hepatic injury and improves organ blood flow during sepsis. Surgery 130,339-345 (2001).
5. Weaver, J. G., Rouse, M. S., Steckelberg, J. M. & Badley,A. D. Improved survival in experimental sepsis with an orally administered inhibitor of apoptosis. FASEB J. 18, 1185-1191 (2004).
6. Wesche-Soldato, D. E. et al .In vivo delivery of caspase 8 or Fas siRNA improves the survival of septic mice. Blood 106, 2295-2301 (2005).
7. White, K. et al. Genetic control of programmed cell death in Drosophila. Science 264, 677-683 (1994).
8. White, K., Tahaoglu, E. & Steller,H. Cell killing by the Drosophila gene reaper. Science 271,805-807 (1996).
9. Ayala,A,Xin Xu, Y., Ayala, C.A., Sonefeld, D.E., Karr, S.M., Evans, T.A.& Chaudry, I.H. Increased mucosal B-lymphocyte apoptosis during polymicrobial sepsis is a Fas ligand but not an endotoxin-mediated process. Blood 91, 1362-1372 (1998).
10. Parker, S. J. & Watldns, P. E. Experimental models of gram-negative sepsis. Br. J. Surg. 88, 22-30 (2001).
11. Wichterman, K. A., Baue, A. E. & Chaudry, I. H. Sepsis and septic shock-a review of laboratory models and a proposal. J. Surg. Res. 29, 189-201 (1980).
12. Kyncl, J.J. et aL Terazosin, a new quinazoline antihypertensive agent. I. General phannacology In: Focus on Alpha Blockade and Terazosin, edited by J. Rosenthal, Zuckschwerdt Verlag Munich (1985).
13. Cavero, I., Lefevre, F. & Roach, A.G. Differential effects of prazosin on the pre- and postsynaptic a-adrenoceptors in the rat and dog. Br. J. Pharmac. 61, 469P (1977).
14. McCall, K, & Steller, H. Facing death in the fly: genetic analysis of apoptosis in Drosophila. Trends Genet 13, 222-226 (1997).
15. Mazzoni et al., PGK1, the gene encoding the glycolitic enzyme phosphoglycerate kinase, acts as a mutilcopy suppressor of apoptotic phenotypes in S. cerevisiae. Yeast 26: 31-37 (2009).
16. Lamb, J. et al. The Connectivity Map: using gene-expression signatures to connect small molecules, genes, and disease. Science 313, 1929-1935 (2006).

## Claims

1. The compound of Formula I , or a pharmaceutical acceptable salt, solvate, ester, or prodrug thereof: 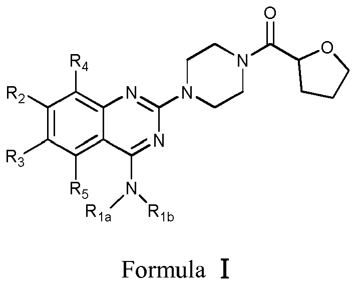 wherein,
R₁ₐ and R_{1b} are independently selected from H, NH₂, OH, C₁₋₆ alkyl, C₁₋₆ alkoxy -C₁₋₆ alkyl-, C₂₋₆ alkenyl, C₂₋₆ alkynyl, C₁₋₆ alkoxy, C₁₋₆ alkanoyl, aroyl, C₆₋₁₀ aryl, and C₅₋₆ cycloalkyl, or R₁ₐ and R_{1b} together with the nitrogen atom to which they are attached form a 5- or 6-membered ring, wherein said alkyl is optionally substituted with 1 to 3 substituents each independently selected from hydroxyl and a halogen;
R₂ and R₃ are independently selected from H, a halogen, C₁₋₆ alkyl, C₁₋₆ haloalkyl, C₂₋₆ alkenyl, C₂₋₆ alkynyl, CN, NO₂, NH₂, OH, C₁₋₆ alkoxy, C₁₋₆ alkoxy-C₁₋₆ alkoxy-, C₁₋₆ alkanoyloxy, C₁₋₆ alkanoylamino, aroylamino, and saturated or unsaturated 5- or 6-membered carbocyclyl or heterocyclyl, C₁₋₆ alkanoyl, or R₂ and R₃ together with the ring atoms to which they are attached form a 5- or 6-membered carbocyclic ring or heterocyclic ring;
R₄ and R₅ are independently selected from H, a halogen, CN, NO₂, NH₂, OH, C₁₋₆ alkyl, C₁₋₆ alkoxy-C₁₋₆ alkoxy, C₁₋₆ alkanoyloxy, C₁₋₆ haloalkyl, C₂₋₆ alkenyl, C₂₋₆ alkynyl, C₁₋₆ alkoxy, C₁₋₆ alkanoylamino, aroylamino, saturated or unsaturated 5- or 6- membered carbocyclyl or heterocyclyl, saturated or unsaturated 5- or 6- membered carbocyclyloxy or heterocyclyloxy, or C₁₋₆ alkanoyl.

2. The compound of claim 1, wherein R₁ₐ and R_{1b} are independently selected from H, NH₂, OH, C₁₋₆ alkyl, C₁₋₄ alkoxy -C₁₋₄ alkyl-, C₂₋₄ alkenyl, C₂₋₄ alkynyl, C₁₋₄ alkoxy, C₁₋₄ alkanoyl, benzoyl, phenyl, C₅₋₆ cycloalkyl, or R₁ₐ and R_{1b} together with the nitrogen atom to which they are attached form a 5- or 6-membered ring, wherein the alkyl is optionally substituted with 1 to 3 substituents each independently selected from hydroxyl or a halogen.

3. The compound of claim 1, wherein R₂ and R₃ are independently selected from H, a halogen, C₁₋₆ alkyl, C₁₋₆ haloalkyl, C₁₋₆ alkoxy, C₁₋₆ alkoxy-C₁₋₆ alkoxy, C₁₋₆ alkanoyloxy, C₁₋₆ alkanoylamino, aroylamino, and saturated or unsaturated 5- or 6-membered carbocyclyl or heterocyclyl, or R₂ and R₃ together with the ring atom to which they are attached form a 5- or 6-membered carbocyclic ring or heterocyclic ring.

4. The compound of claim 1, wherein R₄ and R₅ are independently selected from H, a halogen, C₁₋₆ alkyl, C₁₋₆ alkoxy-C₁₋₆ alkoxy, C₁₋₆ alkanoyloxy, C₁₋₆ haloalkyl, C₁₋₆ alkoxy, C₁₋₆ alkanoylamino, aroylamino, saturated or unsaturated 5- or 6-membered carbocyclyl or heterocyclyl, and saturated or unsaturated 5- or 6- membered carbocyclyloxy or heterocyclyloxy.

5. The compound of claim 1, the compound of Formula I is selected from the compounds numbered Co.1 to Co.33:
| **NO.** | **R₁ₐ** | **R_{1b}** | **R₂** | **R₃** | **R₄** | **R₅** | **Activity*** |
|---|---|---|---|---|---|---|---|
| **Co.1** | CH₃C(O)- | H | CH₃O- | CH₃O- | H | H | 2.9 |
| **Co.2** | -(-CH₂)₂-O-(CH₂)₂-OH | H | CH₃O- | CH₃O- | H | H | 2.3 |
| **Co.3** | -NH₂ | H | CH₃O- | CH₃O- | H | H | 1.9 |
| **Co.4** | -OH | H | CH₃O- | CH₃O- | H | H | 3.4 |
| **Co.5** | -CH₂-CH=CH₂ | H | CH₃O- | CH₃O- | H | H | 1.5 |
| **Co.6** | -CH₂C≡CH | H | CH₃O- | CH₃O- | H | H | 1.2 |
| **Co.7** | -(CH₂)₅CH₃ | H | CH₃O- | CH₃O- | H | H | 2.1 |
| **Co.8** | -(CH₂)₄-CF₃ | H | CH₃O- | CH₃O- | H | H | 0.9 |
| **Co.9** | cyclohexyl | H | CH₃O- | CH₃O- | H | H | 1.4 |
| **Co.10** | -CH₂-(CH₂)₃-CH₂- | | CH₃O- | CH₃O- | H | H | 0.7 |
| **Co.11** | -(CH₂)₂-O-(CH₂)₂-OH | CH₃ | CH₃O- | CH₃O- | H | H | 2.1 |
| **Co.12** | -Ph | H | CH₃O- | CH₃O- | H | H | 2.6 |
| **Co.13** | -C(O)-CF₃ | H | CH₃O- | CH₃O- | H | H | 1.8 |
| **Co.14** | -C(O)-Ph | H | CH₃O- | CH₃O- | H | H | 1.5 |
| **Co.15** | H | H | -CH₂-O-CH₂- | | H | H | 0.6 |
| **Co.16** | H | H | -O(CH₂)₂-O-C₂H₅ | -O(CH₂)₂-O-C₂H₅ | H | H | 1.7 |
| **Co.17** | H | H | -OC(O)CH₃ | -OCH₃ | H | H | 1.1 |
| **Co.18** | H | H | -F | -OCH₃ | H | H | 1.3 |
| **Co.19** | H | H | 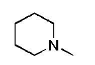 | -CF₃ | H | H | 1.4 |
| **Co.20** | H | H | 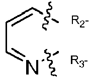 | | H | H | 1.9 |
| **Co.21** | H | H | -NHCOCH₃ | -OCH₃ | H | H | 2.2 |
| **Co.22** | H | H | -(CH₂)₂CH₃ | -OCH₃ | H | H | 2.4 |
| **Co.23** | H | H | -NHCOPh | -OCH₃ | H | H | 0.7 |
| **Co.24** | H | H | 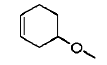 | -OCH₃ | H | H | 0.4 |
| **Co.25** | H | H | -OCH₃ | -OCH₃ | -O(CH₂)₂-O-C₂H₅ | -O(CH₂)₂-O-C₂H₅ | 2.7 |
| **Co.26** | H | H | -OCH₃ | -OCH₃ | -OC(O)CH₃ | -OCH₃ | 3.2 |
| **Co.27** | H | H | -OCH₃ | -OCH₃ | 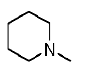 | -CF₃ | 0.9 |
| **Co.28** | H | H | -OCH₃ | -OCH₃ | -F | -OCH₃ | 1.2 |
| **Co.29** | H | H | -OCH₃ | -OCH₃ | -NHCOCH₃ | -OCH₃ | 2.6 |
| **Co.30** | H | H | -OCH₃ | -OCH₃ | -(CH₂)₃CH₃ | -OCH₃ | 1.8 |
| **Co.31** | H | H | -OCH₃ | -OCH₃ | -NHCOPh | -OCH₃ | 0.6 |
| **Co.32** | H | H | -OCH₃ | -OCH₃ | 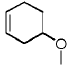 | -OCH₃ | 2.8 |
| **Co.33** | H | H | CH₃O- | CH₃O- | H | H | 2.9 |

6. Use of the compound of any one of claims 1-5 in manufacturing a medicament as an apoptosis inhibitor, as a pgk1 activator, or for the treatment and/or prevention of sepsis and complications thereof.

7. The use of claim 6, wherein the apoptosis inhibitor or the pgk1 activator is used for the treatment and/or prevention of diseases and the diagnosis and/or detection thereof clinically or in a laboratory.

8. The use of claim 6, wherein the sepsis is a caused by bacteria and/or other microbial infections.

9. The use of claim 6, wherein the complication of sepsis is selected from renal failure, respiratory failure, blood clotting disorders, organ damage, toxic cardiomyopathy, encephalopathy, liver disease and toxic intestinal paralysis, purulent meningitis, pneumonia, lung abscess, cellulitis, osteomyelitis, and pyelonephritis.

10. The use of claim 6, wherein the compound of Formula I is terazosin or pharmaceutically acceptable salt or solvate thereof.

11. The use of claim 6, wherein the medicament also further comprises at least one antimicrobial agent.

12. The use of claim 11, wherein the antimicrobial agent is selected from amoxicillin, penicillin, penicillinV, oxacillin, cloxacillin, flucloxacillin, ampicillin, piperacillin, azlocillin, potassium clavulanate, sulbactam, sultamicillin, tazobactam, aztreonam, and meropenem.

13. A pharmaceutical composition comprising a therapeutically and/or prophylactically effective amount of the compound of any one of claims 1-5, and optionally a pharmaceutically acceptable carrier.

14. The pharmaceutical composition of claim 13, wherein further comprising at least one antimicrobial agent.

15. The pharmaceutical composition of claim 14, wherein the antimicrobial agent is selected from amoxicillin, penicillin, penicillinV, oxacillin, cloxacillin, flucloxacillin, ampicillin, piperacillin, azlocillin, potassium clavulanate, sulbactam, sultamicillin, tazobactam, aztreonam, and meropenem.

16. The pharmaceutical composition of claim 13 is used as an apoptosis inhibitor, used as a pgk1 activator, or used for treatment and/or prevention of sepsis and its complications, or used for the treatment and/or prevention of hyperglycemia or cerebral thrombosis, and their complications.
